# EUROPEAN PATENT SPECIFICATION

(11) **EP 3 561 070 B1**
(45) Date of publication and mention of the grant of the patent: **20.10.2021**
(21) Application number: 17889283.2
(22) Date of filing: 20.12.2017
(51) Int. Cl.: C12Q 1/6886, C12Q 1/689, C12Q 1/6895

(54) **METHOD FOR DIAGNOSING BREAST CANCER VIA MICROBIAL METAGENOMIC ANALYSIS**
VERFAHREN ZUR DIAGNOSE VON BRUSTKREBS DURCH MIKROBIELLE METAGENOMISCHE ANALYSE
PROCÉDÉ DE DIAGNOSTIC DU CANCER DU SEIN PAR ANALYSE MÉTAGÉNOMIQUE MICROBIENNE

(30) Priority: 26.12.2016 KR 20160179247; 13.06.2017 KR 20170074046
(43) Date of publication of application: 30.10.2019
(73) Proprietor: MD Healthcare Inc., Seoul 03923 (KR)
(72) Inventor: KIM, Yoon-Keun, Namyangju-si Gyeonggi-do 12146 (KR)
(74) Representative: Dehns
(86) International application number: PCT/KR2017/015115
(87) International publication number: WO 2018/124606

(56) References cited:
- WO-A1-2014/146202
- WO-A1-2016/085356
- WO-A1-2018/008895
- WO-A1-2018/030732
- KR-A- 20110 025 603
- KR-A- 20160 073 157
- KR-A- 20160 102 820
- KR-A- 20160 107 632
- KR-A- 20160 110 232
- KR-A- 20160 140 565
- KR-B1- 101 445 243
- US-A1- 2013 121 968
- US-A1- 2015 017 664
- Urbaniak, C. et al: "Microbiota of Human Breast Tissue and Its Association with Breast Cancer", Applied and Environmental Microbiology, vol. 82, no. 16, 24 June 2016 (2016-06-24) , pages 5039-5048, XP055602741, ISSN: 0099-2240, DOI: 10.1128/AEM.01235-16

## Description

### [Technical Field]

The present invention relates to a method of diagnosing breast cancer through microbial metagenomic analysis, and more particularly, to a method of diagnosing breast cancer by analyzing an increase or decrease in content of extracellular vesicles derived from specific bacteria and archaea through metagenomic analysis of a microorganism such as bacteria, archaea, or the like using a sample derived from a subject.

### [Background Art]

Breast carcinoma collectively refers to all malignant tumors occurring in the breast. Breast cancer is a fatal disease that continues to grow in abnormal breast tissue or spreads to other organs. Although breast cancer has been researched most among all cancers, there is only indefinite knowledge that breast cancer occurs by two factors: an environmental factor and a genetic factor, and there is still no established theory regarding the causes of the onset of breast cancer. However, according to several research results, there is a consensus that several factors are highly correlated and among these, estrogen, which is a female hormone, plays an important role in the development of breast cancer. The risk factors for causes of the onset of breast cancer have been known, and it was reported that, when parents, a sibling, or a daughter in the family has breast cancer, the risk of developing breast cancer himself or herself increases 1.7 times, particularly, 2.4 times in the case of breast cancer occurring prior to menopause, and 9 times in a case in which two or more of the family members have bilateral breast cancer. According to foreign reports, about 10% of breast cancer patients have inherited breast cancer with breast cancer genes (BRCA-1 and BRCA-2). 60% and 85% of individuals with breast cancer genes develop breast cancer prior to 50 years of age and by 70 years of age, respectively, and 65% may simultaneously develop ovarian cancer by 70 years of age.

In addition, long-term activation of reproductive hormones may lead to breast cancer due to carcinogenic mutations of ductal epithelial cells. In particular, it has been known that women having early menarche (before 12 years old) or late menopause (two-fold after 55 years old), women who did not have a child, women who had the first pregnancy after 30 years old, premenopausal women who have no ability to breastfeed, women who have taken contraceptives for 10 years or longer, and women who have received hormone replacement therapy for 10 years or longer to treat facial flushes occurring in menopause or prevent osteoporosis or heart disease have a risk for developing breast cancer. As other environmental factors, it is known to be correlated with drinking, high-fat diets (particularly, polyunsaturated fats such as corn oil, margarine, and the like and saturated fat of beef), overweight postmenopausal women, radiation exposure, and the like. The age at onset of breast cancer is generally higher than that of benign breast disease, and although the average age is 45 years old for Korean women and 55 years old for American women, which is a 10-year difference, the frequency increases with age.

However, despite the fact that breast cancer is most researched among solid cancers, there is still no method of predicting breast cancer using a non-invasive method, and there are many cases in which solid cancers such as breast cancer and the like are detected after the onset thereof, using existing diagnosis methods. Thus, to reduce medical costs and prevent death due to breast cancer, it is efficient to provide a method of predicting the onset of breast cancer and causative factors thereof and preventing the onset of breast cancer in a high-risk group.

Meanwhile, it is known that the number of microorganisms symbiotically living in the human body is 100 trillion which is 10 times the number of human cells, and the number of genes of microorganisms exceeds 100 times the number of human genes. A microbiota or microbiome is a microbial community that includes bacteria, archaea, and eukaryotes present in a given habitat. The intestinal microbiota is known to play a vital role in human's physiological phenomena and significantly affect human health and diseases through interactions with human cells. Bacteria coexisting in human bodies secrete nanometer-sized vesicles to exchange information about genes, proteins, and the like with other cells. The mucous membranes form a physical barrier membrane that does not allow particles with the size of 200 nm or more to pass therethrough, and thus bacteria symbiotically living in the mucous membranes are unable to pass therethrough, but bacteria-derived extracellular vesicles have a size of approximately 100 nm or less and thus relatively freely pass through the mucous membranes and are absorbed into the human body.

Metagenomics, also called environmental genomics, may be analytics for metagenomic data obtained from samples collected from the environment, and collectively refers to a total genome of all microbiota in the natural environment in which microorganisms exist and was first used by Jo Handelsman in 1998 (Handelsman et al., 1998 Chem. Biol. 5, R245-249). Recently, the bacterial composition of human microbiota has been listed using a method based on 16s ribosomal RNA (16s rRNA) base sequences, and 16s rDNA base sequences, which are genes of 16s ribosomal RNA, are analyzed using a next generation sequencing (NGS) platform.

WO2014146202 relates to bacteria for the prevention, treatment and diagnosis of breast cancer.

In the onset of breast cancer, however, identification of causative factors of breast cancer through metagenomic analysis of microbe-derived vesicles isolated from a human-derived substance, such as blood, urine or the like, and a method of predicting breast cancer have never been reported.

### [Disclosure]

### [Technical Problem]

To diagnose breast cancer, the inventors of the present invention extracted DNA from bacteria- and archaea-derived extracellular vesicles using serum, which is a subject-derived sample, and performed metagenomic analysis on the extracted DNA, and, as a result, identified bacteria-derived extracellular vesicles capable of acting as a causative factor of breast cancer, thus completing the present invention based on these findings.

Therefore, it is an object of the present invention to provide a method of providing information for breast cancer diagnosis by metagenomic analysis of bacteria- and archaea-derived extracellular vesicles.

However, the technical goals of the present invention are not limited to the aforementioned goals, and other unmentioned technical goals will be clearly understood by those of ordinary skill in the art from the following description.

### [Technical Solution]

The present invention is defined in the appended claims. According to an aspect of the present invention, there is provided a method of providing information for breast cancer diagnosis, comprising the following processes:
(a) extracting DNA from extracellular vesicles isolated from a subject sample;
(b) performing polymerase chain reaction (PCR) on the extracted DNA using a pair of primers having SEQ ID NO: 1 and SEQ ID NO: 2; and
(c) comparing an increase or decrease in content of bacteria- and archaea-derived extracellular vesicles of the subject sample with that of a normal individual-derived sample through sequencing of a product of the PCR, as defined in present claim 1.

The present invention also provides a method of diagnosing breast cancer, comprising the following processes:
(a) extracting DNA from extracellular vesicles isolated from a subject sample;
(b) performing polymerase chain reaction (PCR) on the extracted DNA using a pair of primers having SEQ ID NO: 1 and SEQ ID NO: 2; and
(c) comparing an increase or decrease in content of bacteria- and archaea-derived extracellular vesicles of the subject sample with that of a normal individual-derived sample through sequencing of a product of the PCR, as defined in present claim 3.

The present disclosure also provides a method of predicting a risk for breast cancer, comprising the following processes:
(a) extracting DNA from extracellular vesicles isolated from a subject sample;
(b) performing polymerase chain reaction (PCR) on the extracted DNA using a pair of primers having SEQ ID NO: 1 and SEQ ID NO: 2; and
(c) comparing an increase or decrease in content of bacteria- and archaea-derived extracellular vesicles of the subject sample with that of a normal individual-derived sample through sequencing of a product of the PCR.

In one embodiment of the present invention, in process (c), breast cancer may be diagnosed by comparing, between blood samples, an increase or decrease in content of extracellular vesicles derived from one or more bacteria selected from the group consisting of the phylum *Fusobacteria* and the phylum *Bacteroidetes.*

In another embodiment of the present invention, in process (c), breast cancer may be diagnosed by comparing, between blood samples, an increase or decrease in content of extracellular vesicles derived from one or more bacteria selected from the group consisting of the class *Fusobacteriia,* the class *Alphaproteobacteria,* the class *Chloroplast,* the class TM7-3, and the class *Bacteroidia.*

In another embodiment of the present invention, in process (c), breast cancer may be diagnosed by comparing, between blood samples, an increase or decrease in content of extracellular vesicles derived from one or more bacteria selected from the group consisting of the order *Fusobacteriales,* the order *Sphingomonadales,* the order *Neisseriales,* the order *Rhizobiales,* the order *Rhodospirillales,* the order *Actinomycetales,* the order *Bacillales,* the order *Streptophyta,* the order *Caulobacterales,* the order *Pseudomonadales,* the order *Bacteroidales,* the order *Enterobacteriales,* and the order *Bifidobacteriales.*

In another embodiment of the present invention, in process (c), breast cancer may be diagnosed by comparing, between blood samples, an increase or decrease in content of extracellular vesicles derived from one or more bacteria selected from the group consisting of the family *Staphylococcaceae,* the family *Fusobacteriaceae,* the family *Actinomycetaceae,* the family *Brevibacteriaceae,* the family *Peptostreptococcaceae,* the family *Rhizobiaceae,* the family *Corynebacteriaceae,* the family *Methylobacteriaceae,* the family *Propionibacteriaceae,* the family *Sphingomonadaceae,* the family *Neisseriaceae,* the family *Flavobacteriaceae,* the family *Intrasporangiaceae,* the family *Nocardiaceae,* the family *Caulobacteraceae,* the family *Micrococcaceae,* the family *Oxalobacteraceae,* the family *Moraxellaceae,* the family *Pseudomonadaceae,* the family *Porphyromonadaceae,* the family *Clostridiaceae,* the family *Rikenellaceae,* the family *Veillonellaceae,* the family *Enterobacteriaceae,* the family S24-7, the family *Bacteroidaceae,* and the family *Bifidobacteriaceae.*

In another embodiment of the present invention, in process (c), breast cancer may be diagnosed by comparing, between blood samples, an increase or decrease in content of extracellular vesicles derived from one or more bacteria selected from the group consisting of the genus *Hydrogenophilus,* the genus *Staphylococcus,* the genus *Fusobacterium,* the genus *Actinomyces,* the genus *Brevibacterium,* the genus *Granulicatella,* the genus *Neisseria,* the genus *Rothia,* the genus *Corynebacterium,* the genus *Brevundimonas,* the genus *Propionibacterium,* the genus *Porphyromonas,* the genus *Sphingomonas,* the genus *Methylobacterium,* the genus *Micrococcus,* the genus *Coprococcus,* the genus *Rhodococcus,* the genus *Cupriavidus,* the genus *Acinetobacter,* the genus *Pseudomonas,* the genus *Enhydrobacter,* the genus *Ruminococcus,* the genus *Dialister,* the genus *Tepidimonas,* the genus *Veillonella,* the genus *Phascolarctobacterium,* the genus *Lachnospira,* the genus *Klebsiella,* the genus *Roseburia,* the genus *Parabacteroides,* the genus *Bacteroides,* the genus *Bifidobacterium,* the genus *Megamonas,* and the genus *Enterobacter.*

In another embodiment of the present invention, in process (c), breast cancer may be diagnosed by comparing, between urine samples, an increase or decrease in content of extracellular vesicles derived from one or more bacteria selected from the group consisting of the phylum *Tenericutes,* the phylum *Armatimonadetes,* the phylum *Cyanobacteria,* the phylum *Verrucomicrobia,* the phylum TM7, and the phylum *Fusobacteria.*

In another embodiment of the present invention, in process (c), breast cancer may be diagnosed by comparing, between urine samples, an increase or decrease in content of extracellular vesicles derived from one or more bacteria selected from the group consisting of the class *Mollicutes,* the class *Chloroplast,* the class *Fimbriimonadia,* the class TM7-3, the class *Verrucomicrobiae,* the class *Saprospirae,* the class *Fusobacteriia,* and the class 4C0d-2.

In another embodiment of the present invention, in process (c), breast cancer may be diagnosed by comparing, between urine samples, an increase or decrease in content of extracellular vesicles derived from one or more bacteria selected from the group consisting of the order *Stramenopiles,* the order RF39, the order *Streptophyta,* the order *Fimbriimonadales,* the order *Verrucomicrobiales,* the order *Saprospirales,* the order *Pseudomonadales,* the order *Fusobacteriales,* the order *Burkholderiales,* the order *Bifidobacteriales,* the order *Oceanospirillales,* and the order YS2.

In another embodiment of the present invention, in process (c), breast cancer may be diagnosed by comparing, between urine samples, an increase or decrease in content of extracellular vesicles derived from one or more bacteria selected from the group consisting of the family *Exiguobacteraceae,* the family *Cellulomonadaceae,* the family F16, the family *Fimbriimonadaceae,* the family *Oxalobacteraceae,* the family *Streptomycetaceae,* the family *Carnobacteriaceae,* the family *Actinomycetaceae,* the family *Nocardiaceae,* the family *Peptococcaceae,* the family *Fusobacteriaceae,* the family *Mogibacteriaceae,* the family *Pseudomonadaceae,* the family *Verrucomicrobiaceae,* the family *Bradyrhizobiaceae,* the family *Enterococcaceae,* the family *Chitinophagaceae,* the family *Moraxellaceae,* the family *Rhizobiaceae,* the family *Ruminococcaceae,* the family *Bacteroidaceae,* the family *Dermacoccaceae,* the family *Gordoniaceae,* the family *Acetobacteraceae,* the family *Bifidobacteriaceae,* the family *Comamonadaceae,* the family *Barnesiellaceae,* the family *Peptostreptococcaceae,* the family *Halomonadaceae,* the family *Rikenellaceae,* and the family *Methylocystaceae.*

In another embodiment of the present invention, in process (c), breast cancer may be diagnosed by comparing, between urine samples, an increase or decrease in content of extracellular vesicles derived from one or more bacteria selected from the group consisting of the genus *Ralstonia,* the genus *Rhizobium,* the genus *Morganella,* the genus *Tetragenococcus,* the genus *Exiguobacterium,* the genus *Streptomyces,* the genus *Oribacterium,* the genus *Sporosarcina,* the genus *Jeotgalicoccus,* the genus *Fimbriimonas,* the genus *Enterococcus,* the genus *Porphyromonas,* the genus *Lactococcus,* the genus *Cellulomonas,* the genus *Proteus,* the genus *Granulicatella,* the genus *Acinetobacter,* the genus *Actinomyces,* the genus *Cupriavidus,* the genus *Rhodococcus,* the genus *Fusobacterium,* the genus *Pseudomonas,* the genus *Akkermansia,* the genus *Leptotrichia,* the genus *Methylobacterium,* the genus *Weissella,* the genus *Bacteroides,* the genus *Veillonella,* the genus *Dermacoccus,* the genus *Coprococcus,* the genus *Ruminococcus,* the genus *Trabulsiella,* the genus *Gordonia,* the genus *Lachnospira,* the genus *Klebsiella,* the genus *Enterobacter,* the genus *Faecalibacterium,* the genus *Serratia,* the genus *Bifidobacterium,* the genus *Citrobacter,* the genus *Bilophila,* the genus *Virgibacillus,* the genus *Halomonas,* the genus *Roseburia,* the genus *Comamonas,* the genus *Methylopila,* and the genus *Gemella.*

In the present invention, the subject sample is blood or urine.

In another embodiment of the present invention, the blood may be whole blood, serum, or plasma.

### [Advantageous Effects]

Extracellular vesicles secreted from bacteria and archaea present in the environment are absorbed into the human body, and thus may directly affect the occurrence of cancer, and it is difficult to diagnose breast cancer early before symptoms occur, and thus efficient treatment therefor is difficult. Thus, according to the present invention, a risk of developing breast cancer can be predicted through metagenomic analysis of bacteria-derived extracellular vesicles by using a human body-derived sample, and thus the onset of breast cancer can be delayed or breast cancer can be prevented through appropriate management by early diagnosis and prediction of a risk group for breast cancer, and, even after breast cancer occurs, early diagnosis for breast cancer can be implemented, thereby lowering the incidence rate of breast cancer and increasing therapeutic effects. In addition, patients diagnosed with breast cancer are able to avoid exposure to causative factors predicted by metagenomic analysis, whereby the progression of cancer can be ameliorated, or recurrence of breast cancer can be prevented.

### [Description of Drawings]

FIG. 1A illustrates images showing the distribution pattern of bacteria and extracellular vesicles over time after intestinal bacteria and bacteria-derived extracellular vesicles (EVs) were orally administered to mice, and FIG. 1B illustrates images showing the distribution pattern of bacteria and EVs after being orally administered to mice and, at 12 hours, blood and various organs were extracted.
FIG. 2 illustrates distribution results of bacteria-derived EVs exhibiting significant diagnostic performance at a phylum level, after metagenomic analysis of bacteria-derived EVs isolated from breast cancer patient-derived blood and normal individual-derived blood.
FIG. 3 illustrates distribution results of bacteria-derived EVs exhibiting significant diagnostic performance at a class level, after metagenomic analysis of bacteria-derived EVs isolated from breast cancer patient-derived blood and normal individual-derived blood.
FIG. 4 illustrates distribution results of bacteria-derived EVs exhibiting significant diagnostic performance at an order level, after metagenomic analysis of bacteria-derived EVs isolated from breast cancer patient-derived blood and normal individual-derived blood.
FIG. 5 illustrates distribution results of bacteria-derived EVs exhibiting significant diagnostic performance at a family level, after metagenomic analysis of bacteria-derived EVs isolated from breast cancer patient-derived blood and normal individual-derived blood.
FIG. 6 illustrates distribution results of bacteria-derived EVs exhibiting significant diagnostic performance at a genus level, after metagenomic analysis of bacteria-derived EVs isolated from breast cancer patient-derived blood and normal individual-derived blood.
FIG. 7 illustrates distribution results of bacteria-derived EVs exhibiting significant diagnostic performance at a phylum level, after metagenomic analysis of bacteria-derived EVs isolated from breast cancer patient-derived urine and normal individual-derived urine.
FIG. 8 illustrates distribution results of bacteria-derived EVs exhibiting significant diagnostic performance at a class level, after metagenomic analysis of bacteria-derived EVs isolated from breast cancer patient-derived urine and normal individual-derived urine.
FIG. 9 illustrates distribution results of bacteria-derived EVs exhibiting significant diagnostic performance at an order level, after metagenomic analysis of bacteria-derived EVs isolated from breast cancer patient-derived urine and normal individual-derived urine.
FIG. 10 illustrates distribution results of bacteria-derived EVs exhibiting significant diagnostic performance at a family level, after metagenomic analysis of bacteria-derived EVs isolated from breast cancer patient-derived urine and normal individual-derived urine.
FIG. 11 illustrates distribution results of bacteria-derived EVs exhibiting significant diagnostic performance at a genus level, after metagenomic analysis of bacteria-derived EVs isolated from breast cancer patient-derived urine and normal individual-derived urine.

### [Best Mode]

The present invention relates to a method of diagnosing breast cancer through bacterial and archaeal metagenomic analysis. The inventors of the present invention extracted genes from bacteria- and archaea-derived extracellular vesicles using a subject-derived sample, performed metagenomic analysis thereon, and identified bacteria-derived extracellular vesicles capable of acting as a causative factor of breast cancer.

Thus, the present invention provides a method of providing information for breast cancer diagnosis, the method comprising:
(a) extracting DNA from extracellular vesicles isolated from a subject sample;
(b) performing polymerase chain reaction (PCR) on the extracted DNA using a pair of primers having SEQ ID NO: 1 and SEQ ID NO: 2; and
(c) comparing an increase or decrease in content of bacteria- and archaea-derived extracellular vesicles of the subject sample with that of a normal individual-derived sample through sequencing of a product of the PCR, as defined in claim 1.

The term "breast cancer diagnosis" as used herein refers to determining whether a patient has a risk for breast cancer, whether the risk for breast cancer is relatively high, or whether breast cancer has already occurred. The method of the present disclosure may be used to delay the onset of breast cancer through special and appropriate care for a specific patient, which is a patient having a high risk for breast cancer or prevent the onset of breast cancer. In addition, the method may be clinically used to determine treatment by selecting the most appropriate treatment method through early diagnosis of breast cancer.

The term "metagenome" as used herein refers to the total of genomes including all viruses, bacteria, fungi, and the like in isolated regions such as soil, the intestines of animals, and the like, and is mainly used as a concept of genomes that explains identification of many microorganisms at once using a sequencer to analyze non-cultured microorganisms. In particular, a metagenome does not refer to a genome of one species, but refers to a mixture of genomes, including genomes of all species of an environmental unit. This term originates from the view that, when defining one species in a process in which biology is advanced into omics, various species as well as existing one species functionally interact with each other to form a complete species. Technically, it is the subject of techniques that analyzes all DNAs and RNAs regardless of species using rapid sequencing to identify all species in one environment and verify interactions and metabolism. In the present invention, bacterial metagenomic analysis is performed using bacteria-derived extracellular vesicles isolated from, for example, blood and urine.

In the present invention, the subject sample is blood or urine, and the blood may be whole blood, serum, or plasma.

In an embodiment of the present invention, metagenomic analysis is performed on the bacteria- and archaea-derived extracellular vesicles, and bacteria-derived extracellular vesicles capable of acting as a cause of the onset of breast cancer were actually identified by analysis at phylum, class, order, family, and genus levels.

More particularly, in one embodiment of the present invention, as a result of performing bacterial metagenomic analysis on extracellular vesicles present in subject-derived blood samples at a phylum level, the content of extracellular vesicles derived from bacteria belonging to the phylum *Fusobacteria* and the phylum *Bacteroidetes* was significantly different between breast cancer patients and normal individuals (see Example 4).

More particularly, in one embodiment of the present invention, as a result of performing bacterial metagenomic analysis on extracellular vesicles present in subject-derived blood samples at a class level, the content of extracellular vesicles derived from bacteria belonging to the class *Fusobacteriia,* the class *Alphaproteobacteria,* the class *Chloroplast,* the class TM7-3, and the class *Bacteroidia* was significantly different between breast cancer patients and normal individuals (see Example 4).

More particularly, in one embodiment of the present invention, as a result of performing bacterial metagenomic analysis on extracellular vesicles present in subject-derived blood samples at an order level, the content of extracellular vesicles derived from bacteria belonging to the order *Fusobacteriales,* the order *Sphingomonadales,* the order *Neisseriales,* the order *Rhizobiales,* the order *Rhodospirillales,* the order *Actinomycetales,* the order *Bacillales,* the order *Streptophyta,* the order *Caulobacterales,* the order *Pseudomonadales,* the order *Bacteroidales,* the order *Enterobacteriales,* and the order *Bifidobacteriales* was significantly different between breast cancer patients and normal individuals (see Example 4).

More particularly, in one embodiment of the present invention, as a result of performing bacterial metagenomic analysis on extracellular vesicles present in subject-derived blood samples at a family level, the content of extracellular vesicles derived from bacteria belonging to the family *Staphylococcaceae,* the family *Fusobacteriaceae,* the family *Actinomycetaceae,* the family *Brevibacteriaceae,* the family *Peptostreptococcaceae,* the family *Rhizobiaceae,* the family *Corynebacteriaceae,* the family *Methylobacteriaceae,* the family *Propionibacteriaceae,* the family *Sphingomonadaceae,* the family *Neisseriaceae,* the family *Flavobacteriaceae,* the family *Intrasporangiaceae,* the family *Nocardiaceae,* the family *Caulobacteraceae,* the family *Micrococcaceae,* the family *Oxalobacteraceae,* the family *Moraxellaceae,* the family *Pseudomonadaceae,* the family *Porphyromonadaceae,* the family *Clostridiaceae,* the family *Rikenellaceae,* the family *Veillonellaceae,* the family *Enterobacteriaceae,* the family S24-7, the family *Bacteroidaceae,* and the family *Bifidobacteriaceae* was significantly different between breast cancer patients and normal individuals (see Example 4).

More particularly, in one embodiment of the present invention, as a result of performing bacterial metagenomic analysis on extracellular vesicles present in subject-derived blood samples at a genus level, the content of extracellular vesicles derived from bacteria belonging to the genus *Hydrogenophilus,* the genus *Staphylococcus,* the genus *Fusobacterium,* the genus *Actinomyces,* the genus *Brevibacterium,* the genus *Granulicatella,* the genus *Neisseria,* the genus *Rothia,* the genus *Corynebacterium,* the genus *Brevundimonas,* the genus *Propionibacterium,* the genus *Porphyromonas,* the genus *Sphingomonas,* the genus *Methylobacterium,* the genus *Micrococcus,* the genus *Coprococcus,* the genus *Rhodococcus,* the genus *Cupriavidus,* the genus *Acinetobacter,* the genus *Pseudomonas,* the genus *Enhydrobacter,* the genus *Ruminococcus,* the genus *Dialister,* the genus *Tepidimonas,* the genus *Veillonella,* the genus *Phascolarctobacterium,* the genus *Lachnospira,* the genus *Klebsiella,* the genus *Roseburia,* the genus *Parabacteroides,* the genus *Bacteroides,* the genus *Bifidobacterium,* the genus *Megamonas,* and the genus *Enterobacter* was significantly different between breast cancer patients and normal individuals (see Example 4).

More particularly, in one embodiment of the present invention, as a result of performing bacterial metagenomic analysis on extracellular vesicles present in subject-derived urine samples at a phylum level, the content of extracellular vesicles derived from bacteria belonging to the phylum *Tenericutes,* the phylum *Armatimonadetes,* the phylum *Cyanobacteria,* the phylum *Verrucomicrobia,* the phylum TM7, and the phylum *Fusobacteria* was significantly different between breast cancer patients and normal individuals (see Example 5).

More particularly, in one embodiment of the present invention, as a result of performing bacterial metagenomic analysis on extracellular vesicles present in subject-derived urine samples at a class level, the content of extracellular vesicles derived from bacteria belonging to the class *Mollicutes,* the class *Chloroplast,* the class *Fimbriimonadia,* the class TM7-3, the class *Verrucomicrobiae,* the class *Saprospirae,* the class *Fusobacteriia,* and the class 4C0d-2 was significantly different between breast cancer patients and normal individuals (see Example 5).

More particularly, in one embodiment of the present invention, as a result of performing bacterial metagenomic analysis on extracellular vesicles present in subject-derived urine samples at an order level, the content of extracellular vesicles derived from bacteria belonging to the order *Stramenopiles,* the order RF39, the order *Streptophyta,* the order *Fimbriimonadales,* the order *Verrucomicrobiales,* the order *Saprospirales,* the order *Pseudomonadales,* the order *Fusobacteriales,* the order *Burkholderiales,* the order *Bifidobacteriales,* the order *Oceanospirillales,* and the order YS2 was significantly different between breast cancer patients and normal individuals (see Example 5).

More particularly, in one embodiment of the present invention, as a result of performing bacterial metagenomic analysis on extracellular vesicles present in subject-derived urine samples at a family level, the content of extracellular vesicles derived from bacteria belonging to the family *Exiguobacteraceae,* the family *Cellulomonadaceae,* the family F16, the family *Fimbriimonadaceae,* the family *Oxalobacteraceae,* the family *Streptomycetaceae,* the family *Carnobacteriaceae,* the family *Actinomycetaceae,* the family *Nocardiaceae,* the family *Peptococcaceae,* the family *Fusobacteriaceae,* the family *Mogibacteriaceae,* the family *Pseudomonadaceae,* the family *Verrucomicrobiaceae,* the family *Bradyrhizobiaceae,* the family *Enterococcaceae,* the family *Chitinophagaceae,* the family *Moraxellaceae,* the family *Rhizobiaceae,* the family *Ruminococcaceae,* the family *Bacteroidaceae,* the family *Dermacoccaceae,* the family *Gordoniaceae,* the family *Acetobacteraceae,* the family *Bifidobacteriaceae,* the family *Comamonadaceae,* the family *Barnesiellaceae,* the family *Peptostreptococcaceae,* the family *Halomonadaceae,* the family *Rikenellaceae,* and the family *Methylocystaceae* was significantly different between breast cancer patients and normal individuals (see Example 5).

More particularly, in one embodiment of the present invention, as a result of performing bacterial metagenomic analysis on extracellular vesicles present in subject-derived urine samples at a genus level, the content of extracellular vesicles derived from bacteria belonging to the genus *Ralstonia,* the genus *Rhizobium,* the genus *Morganella,* the genus *Tetragenococcus,* the genus *Exiguobacterium,* the genus *Streptomyces,* the genus *Oribacterium,* the genus *Sporosarcina,* the genus *Jeotgalicoccus,* the genus *Fimbriimonas,* the genus *Enterococcus,* the genus *Porphyromonas,* the genus *Lactococcus,* the genus *Cellulomonas,* the genus *Proteus,* the genus *Granulicatella,* the genus *Acinetobacter,* the genus *Actinomyces,* the genus *Cupriavidus,* the genus *Rhodococcus,* the genus *Fusobacterium,* the genus *Pseudomonas,* the genus *Akkermansia,* the genus *Leptotrichia,* the genus *Methylobacterium,* the genus *Weissella,* the genus *Bacteroides,* the genus *Veillonella,* the genus *Dermacoccus,* the genus *Coprococcus,* the genus *Ruminococcus,* the genus *Trabulsiella,* the genus *Gordonia,* the genus *Lachnospira,* the genus *Klebsiella,* the genus *Enterobacter,* the genus *Faecalibacterium,* the genus *Serratia,* the genus *Bifidobacterium,* the genus *Citrobacter,* the genus *Bilophila,* the genus *Virgibacillus,* the genus *Halomonas,* the genus *Roseburia,* the genus *Comamonas,* the genus *Methylopila,* and the genus *Gemella* was significantly different between breast cancer patients and normal individuals (see Example 5).

From the above-described example results, it can be confirmed that bacteria-derived extracellular vesicles exhibiting a significant change in content in breast cancer patients compared to normal individuals, are identified by performing bacterial metagenomic analysis on bacteria-derived extracellular vesicles isolated from blood and urine, and breast cancer may be diagnosed by analyzing an increase or decrease in the content of bacteria-derived extracellular vesicles at each level through metagenomic analysis.

Hereinafter, the present invention will be described with reference to exemplary examples to aid in understanding of the present invention. However, these examples are provided only for illustrative purposes and are not intended to limit the scope of the present invention.

### [Examples]

### Example 1. Analysis of In Vivo Absorption, Distribution, and Excretion Patterns of Intestinal Bacteria and Bacteria-Derived Extracellular Vesicles

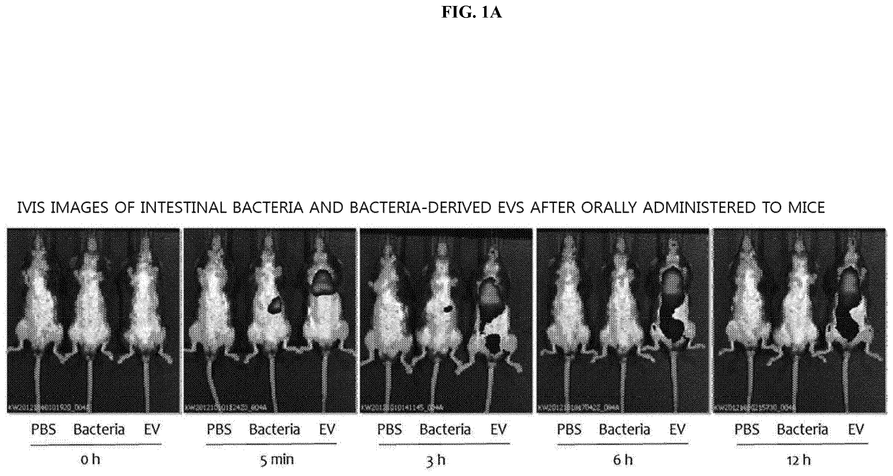

To evaluate whether intestinal bacteria and bacteria-derived extracellular vesicles are systematically absorbed through the gastrointestinal tract, an experiment was conducted using the following method. More particularly, 50 µg of each of intestinal bacteria and the bacteria-derived extracellular vesicles (EVs), labeled with fluorescence, were orally administered to the gastrointestinal tracts of mice, and fluorescence was measured at 0 h, and after 5 min, 3 h, 6 h, and 12 h. As a result of observing the entire images of mice, as illustrated in FIG. 1A, the bacteria were not systematically absorbed when administered, while the bacteria-derived EVs were systematically absorbed at 5 min after administration, and, at 3 h after administration, fluorescence was strongly observed in the bladder, from which it was confirmed that the EVs were excreted via the urinary system, and were present in the bodies up to 12 h after administration.

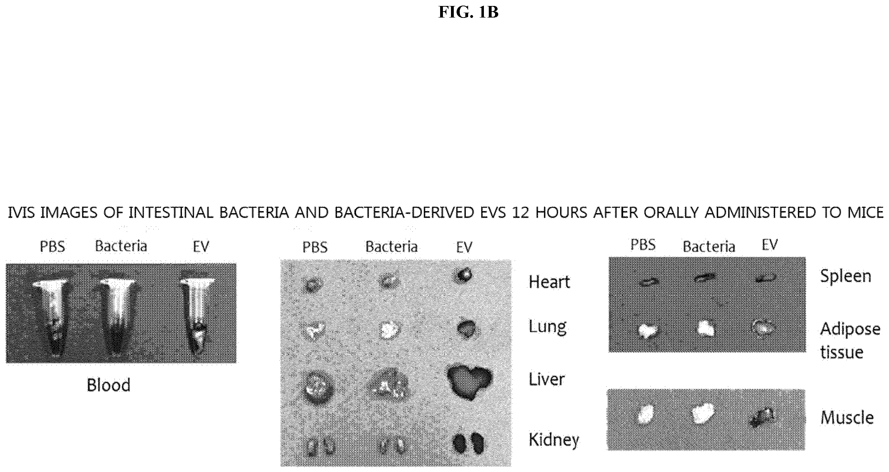

After intestinal bacteria and intestinal bacteria-derived extracellular vesicles were systematically absorbed, to evaluate a pattern of invasion of intestinal bacteria and the bacteria-derived EVs into various organs in the human body after being systematically absorbed, 50 µg of each of the bacteria and bacteria-derived EVs, labeled with fluorescence, were administered using the same method as that used above, and then, at 12 h after administration, blood, the heart, the lungs, the liver, the kidneys, the spleen, adipose tissue, and muscle were extracted from each mouse. As a result of observing fluorescence in the extracted tissues, as illustrated in FIG. 1B, it was confirmed that the intestinal bacteria were not absorbed into each organ, while the bacteria-derived EVs were distributed in the blood, heart, lungs, liver, kidneys, spleen, adipose tissue, and muscle.

### Example 2. Vesicle Isolation and DNA Extraction from Blood and Urine

To isolate extracellular vesicles and extract DNA, from blood and urine, first, blood or urine was added to a 10 ml tube and centrifuged at 3,500 x g and 4 □ for 10 min to precipitate a suspension, and only a supernatant was collected, which was then placed in a new 10 ml tube. The collected supernatant was filtered using a 0.22 µm filter to remove bacteria and impurities, and then placed in centripreigugal filters (50 kD) and centrifuged at 1500 x g and 4 □ for 15 min to discard materials with a smaller size than 50 kD, and then concentrated to 10 ml. Once again, bacteria and impurities were removed therefrom using a 0.22 µm filter, and then the resulting concentrate was subjected to ultra-high speed centrifugation at 150,000 x g and 4 □ for 3 hours by using a Type 90ti rotor to remove a supernatant, and the agglomerated pellet was dissolved with phosphate-buffered saline (PBS), thereby obtaining vesicles.

100 µl of the extracellular vesicles isolated from the blood and urine according to the above-described method was boiled at 100 □ to allow the internal DNA to come out of the lipid and then cooled on ice. Next, the resulting vesicles were centrifuged at 10,000 x g and 4 □ for 30 minutes to remove the remaining suspension, only the supernatant was collected, and then the amount of DNA extracted was quantified using a NanoDrop sprectrophotometer. In addition, to verify whether bacteria-derived DNA was present in the extracted DNA, PCR was performed using 16s rDNA primers shown in Table 1 below.

**[Table 1]**

| Primer | | Sequence | SEQ ID NO. |
|---|---|---|---|
| 16S rDNA | 16S_V3_F | 5'-TCGTCGGCAGCGTC AGATGTGTATAAGAG ACAGCCTACGGGNGG CWGCAG-3' | 1 |
| | 16S_V4_R | 5'-GTCTCGTGGGCTCG GAGATGTGTATAAGA GACAGGACTACHVGG GTATCTAATCC-3' | 2 |

### Example 3. Metagenomic Analysis Using DNA Extracted from Blood and Urine

DNA was extracted using the same method as that used in Example 2, and then PCR was performed thereon using 16S rDNA primers shown in Table 1 to amplify DNA, followed by sequencing (Illumina MiSeq sequencer). The results were output as standard flowgram format (SFF) files, and the SFF files were converted into sequence files (.fasta) and nucleotide quality score files using GS FLX software (v2.9), and then credit rating for reads was identified, and portions with a window (20 bps) average base call accuracy of less than 99% (Phred score <20) were removed. After removing the low-quality portions, only reads having a length of 300 bps or more were used (Sickle version 1.33), and, for operational taxonomy unit (OTU) analysis, clustering was performed using UCLUST and USEARCH according to sequence similarity. In particular, clustering was performed based on sequence similarity values of 94% for genus, 90% for family, 85% for order, 80% for class, and 75% for phylum, and phylum, class, order, family, and genus levels of each OTU were classified, and bacteria with a sequence similarity of 97% or more were analyzed (QIIME) using 16S DNA sequence databases (108,453 sequences) of BLASTN and GreenGenes.

### Example 4. Breast Cancer Diagnostic Model Based on Metagenomic Analysis of Bacteria-Derived EVs Isolated from Blood

EVs were isolated from blood samples of 96 breast cancer patients and 192 normal individuals, the two groups matched in age and gender, and then metagenomic sequencing was performed thereon using the method of Example 3. For the development of a diagnostic model, first, a strain exhibiting a p value of less than 0.05 between two groups in a t-test and a difference of two-fold or more between two groups was selected, and then an area under curve (AUC), sensitivity, and specificity, which are diagnostic performance indexes, were calculated by logistic regression analysis.

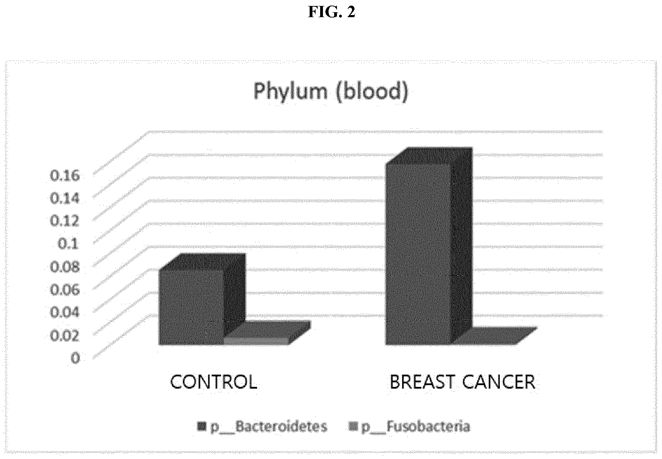

As a result of analyzing bacteria-derived EVs in blood at a phylum level, a diagnostic model developed using bacteria belonging to the phylum *Fusobacteria* and the phylum *Bacteroidetes* as a biomarker exhibited significant diagnostic performance for breast cancer (see Table 2 and FIG. 2).

**[Table 2]**

| | Control | | Breast cancer | | t-test | | Training Set | | | Test Set | | |
|---|---|---|---|---|---|---|---|---|---|---|---|---|
| Taxon | Mea n | SD | Mea n | SD | p-val ue | fold | AU C | sensit ivity | specifi city | AUC | sensiti vity | sp ec ifi cit y |
| p_Fuso bacteria | 0.006 3 | 0.010 1 | 0.001 0 | 0.002 5 | 0.000 0 | 0.16 | 0.7 0 | 0.99 | 0.02 | 0.74 | 0.98 | 0. 06 |
| p_Bact eroidete s | 0.065 4 | 0.035 2 | 0.157 8 | 0.041 9 | 0.000 0 | 2.41 | 0.9 5 | 0.94 | 0.76 | 0.98 | 0.96 | 0. 82 |

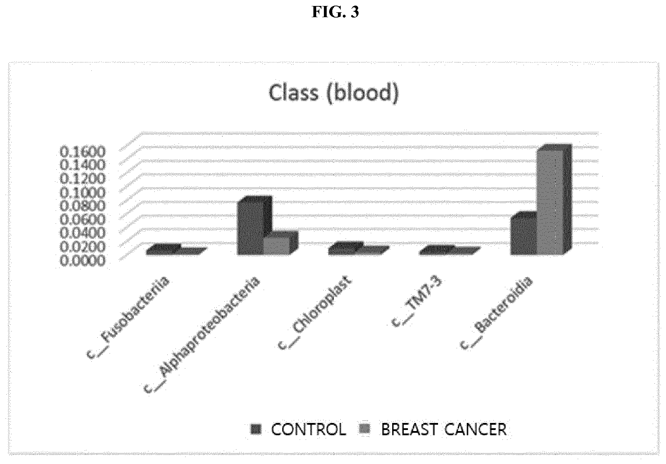

As a result of analyzing bacteria-derived EVs in blood at a class level, a diagnostic model developed using bacteria belonging to the class *Fusobacteriia,* the class *Alphaproteobacteria,* the class *Chloroplast,* the class TM7-3, and the class *Bacteroidia* as a biomarker exhibited significant diagnostic performance for breast cancer (see Table 3 and FIG. 3).

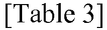

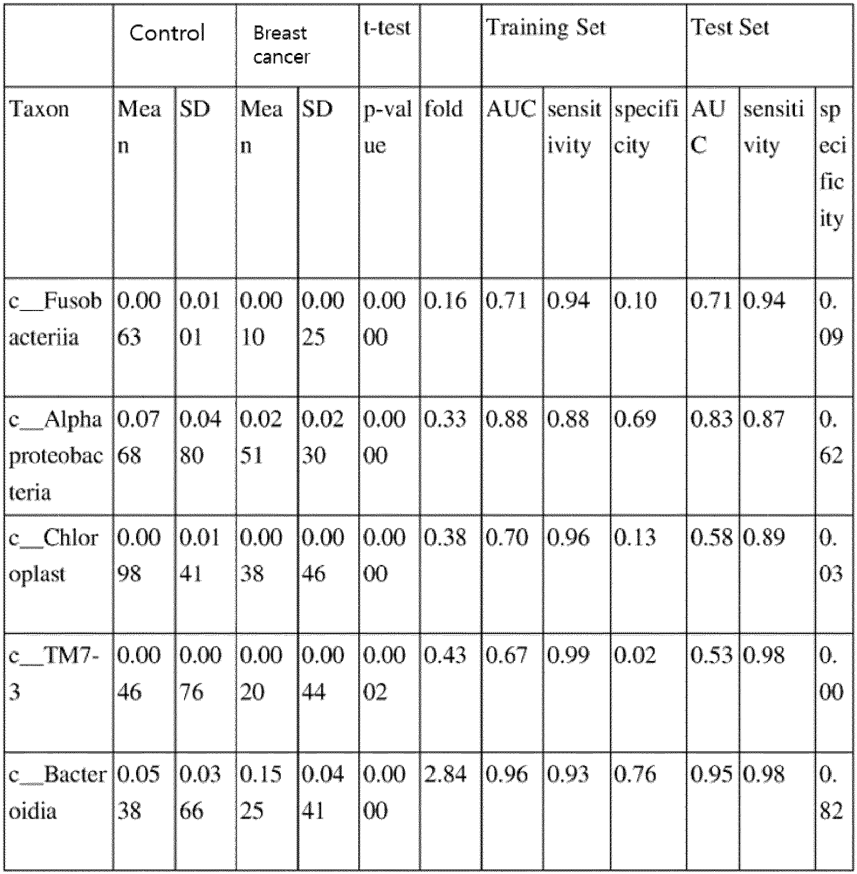

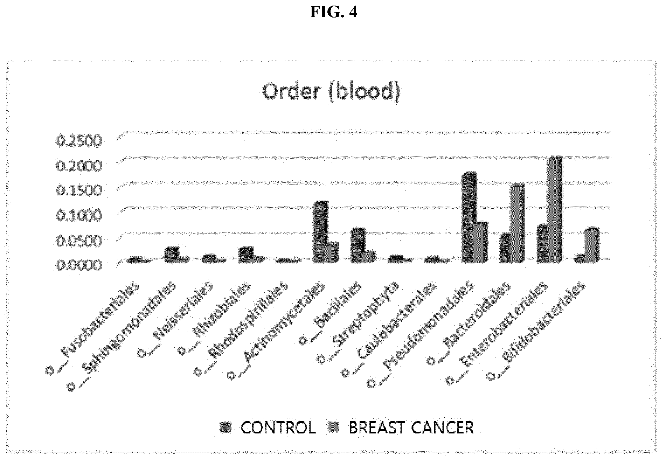

As a result of analyzing bacteria-derived EVs in blood at an order level, a diagnostic model developed using bacteria belonging to the order *Fusobacteriales,* the order *Sphingomonadales,* the order *Neisseriales,* the order *Rhizobiales,* the order *Rhodospirillales,* the order *Actinomycetales,* the order *Bacillales,* the order *Streptophyta,* the order *Caulobacterales,* the order *Pseudomonadales,* the order *Bacteroidales,* the order *Enterobacteriales,* and the order *Bifidobacteriales* as a biomarker exhibited significant diagnostic performance for breast cancer (see Table 4 and FIG. 4).

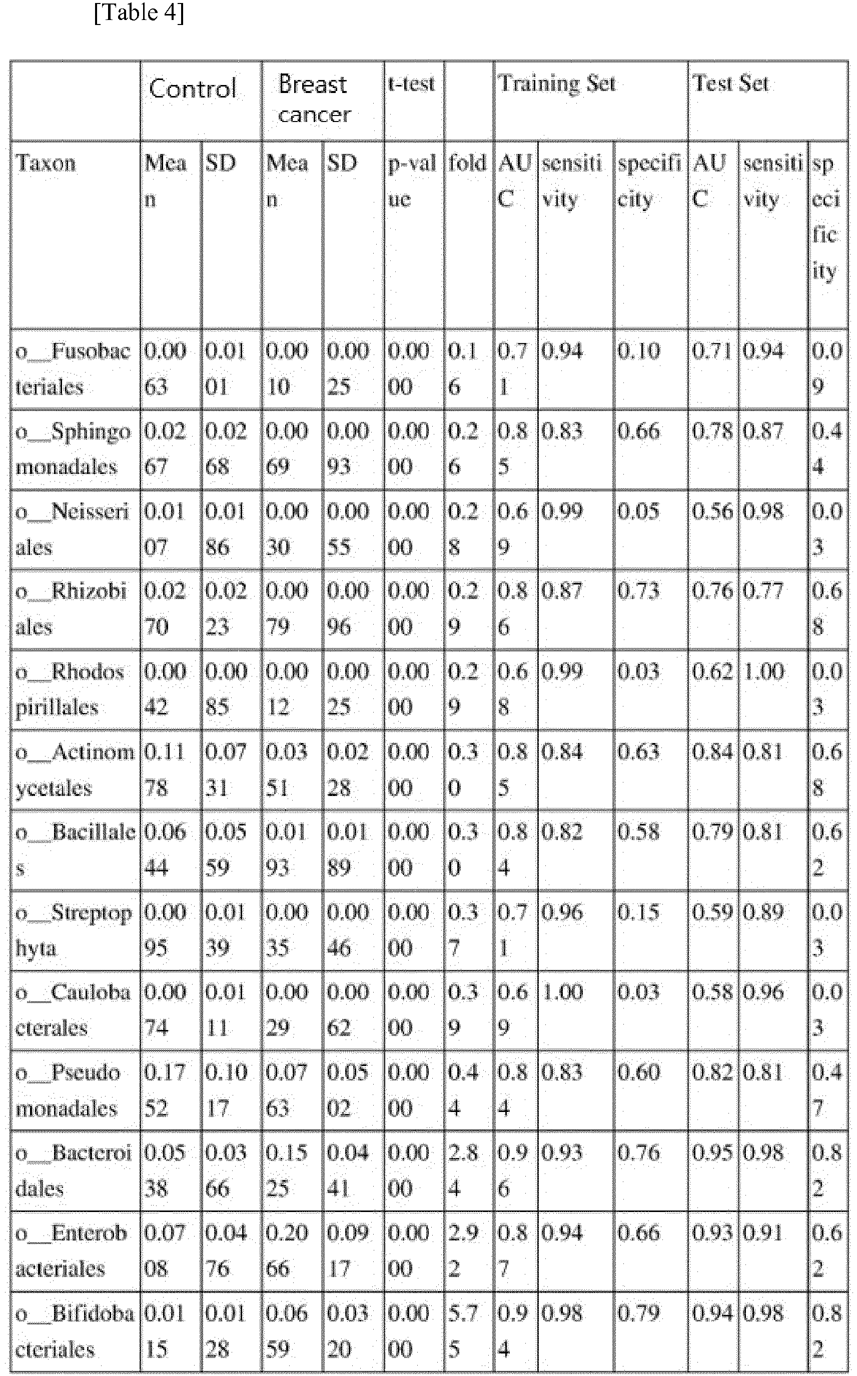

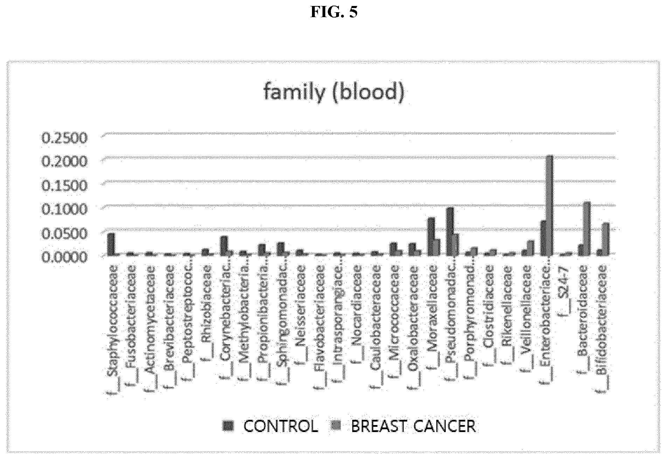

As a result of analyzing bacteria-derived EVs in blood at a family level, a diagnostic model developed using bacteria belonging to the family *Staphylococcaceae,* the family *Fusobacteriaceae,* the family *Actinomycetaceae,* the family *Brevibacteriaceae,* the family *Peptostreptococcaceae,* the family *Rhizobiaceae,* the family *Corynebacteriaceae,* the family *Methylobacteriaceae,* the family *Propionibacteriaceae,* the family *Sphingomonadaceae,* the family *Neisseriaceae,* the family *Flavobacteriaceae,* the family *Intrasporangiaceae,* the family *Nocardiaceae,* the family *Caulobacteraceae,* the family *Micrococcaceae,* the family *Oxalobacteraceae,* the family *Moraxellaceae,* the family *Pseudomonadaceae,* the family *Porphyromonadaceae,* the family *Clostridiaceae,* the family *Rikenellaceae,* the family *Veillonellaceae,* the family *Enterobacteriaceae,* the family S24-7, the family *Bacteroidaceae,* and the family *Bifidobacteriaceae* as a biomarker exhibited significant diagnostic performance for breast cancer (see Table 5 and FIG. 5).

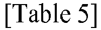

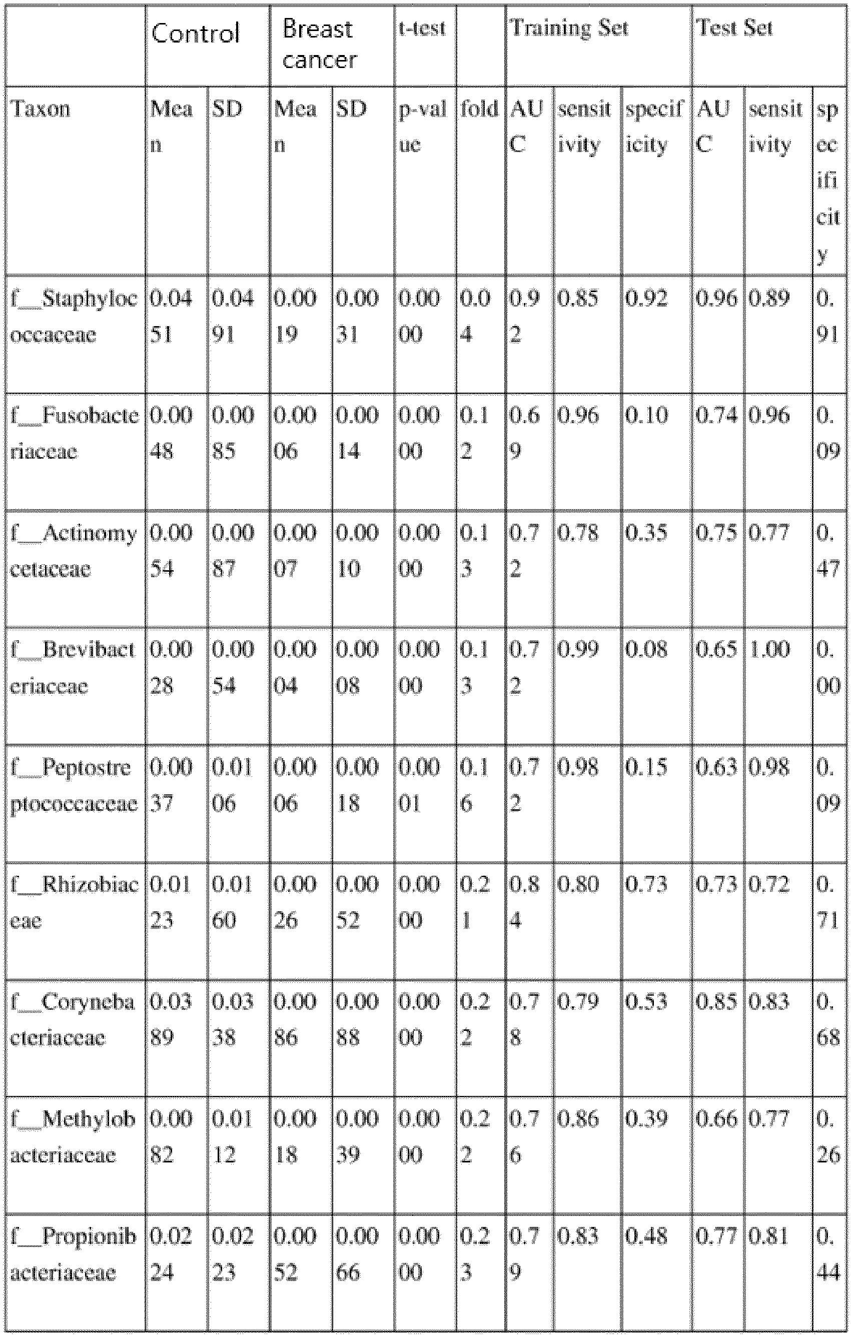

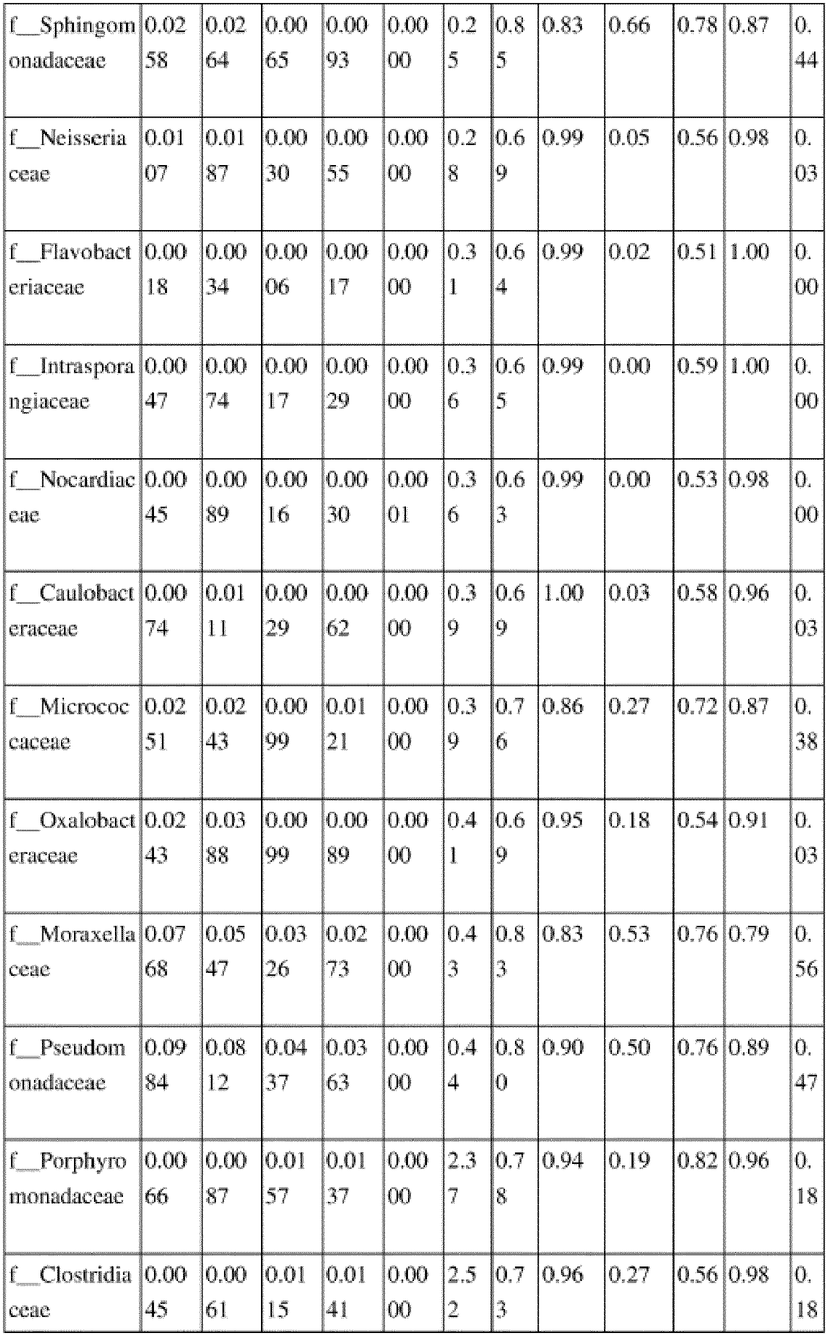

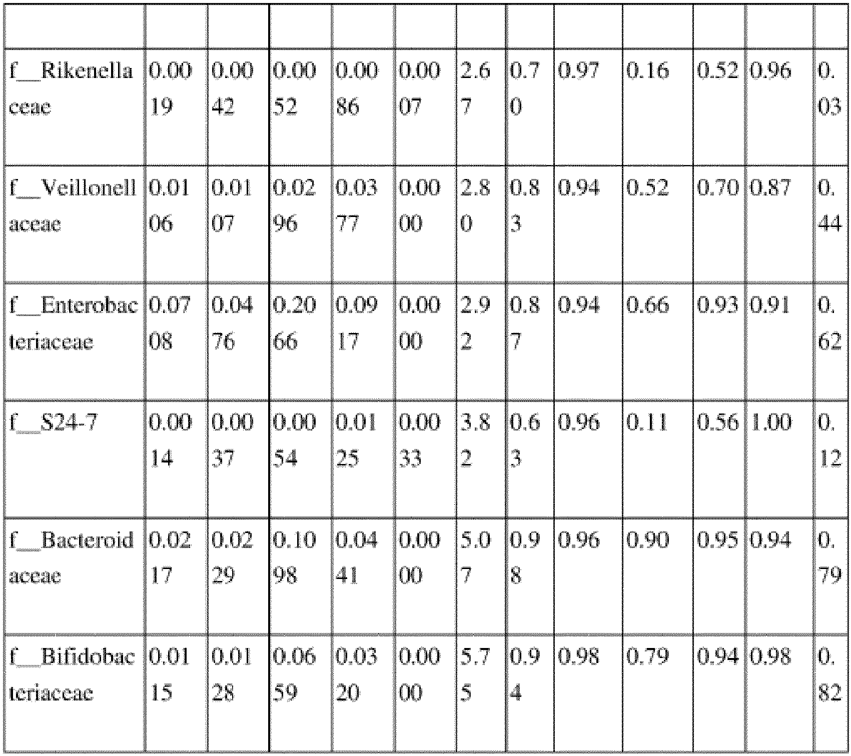

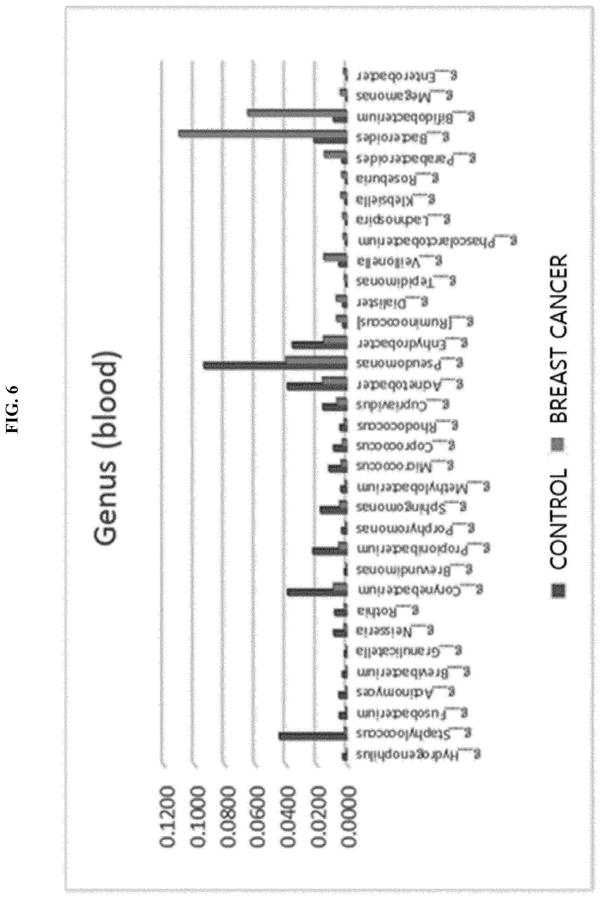

As a result of analyzing bacteria-derived EVs in blood at a genus level, a diagnostic model developed using bacteria belonging to the genus *Hydrogenophilus,* the genus *Staphylococcus,* the genus *Fusobacterium,* the genus *Actinomyces,* the genus *Brevibacterium,* the genus *Granulicatella,* the genus *Neisseria,* the genus *Rothia,* the genus *Corynebacterium,* the genus *Brevundimonas,* the genus *Propionibacterium,* the genus *Porphyromonas,* the genus *Sphingomonas,* the genus *Methylobacterium,* the genus *Micrococcus,* the genus *Coprococcus,* the genus *Rhodococcus,* the genus *Cupriavidus,* the genus *Acinetobacter,* the genus *Pseudomonas,* the genus *Enhydrobacter,* the genus *Ruminococcus,* the genus *Dialister,* the genus *Tepidimonas,* the genus *Veillonella,* the genus *Phascolarctobacterium,* the genus *Lachnospira,* the genus *Klebsiella,* the genus *Roseburia,* the genus *Parabacteroides,* the genus *Bacteroides,* the genus *Bifidobacterium,* the genus *Megamonas,* and the genus *Enterobacter* as a biomarker exhibited significant diagnostic performance for breast cancer (see Table 6 and FIG. 6).

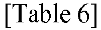

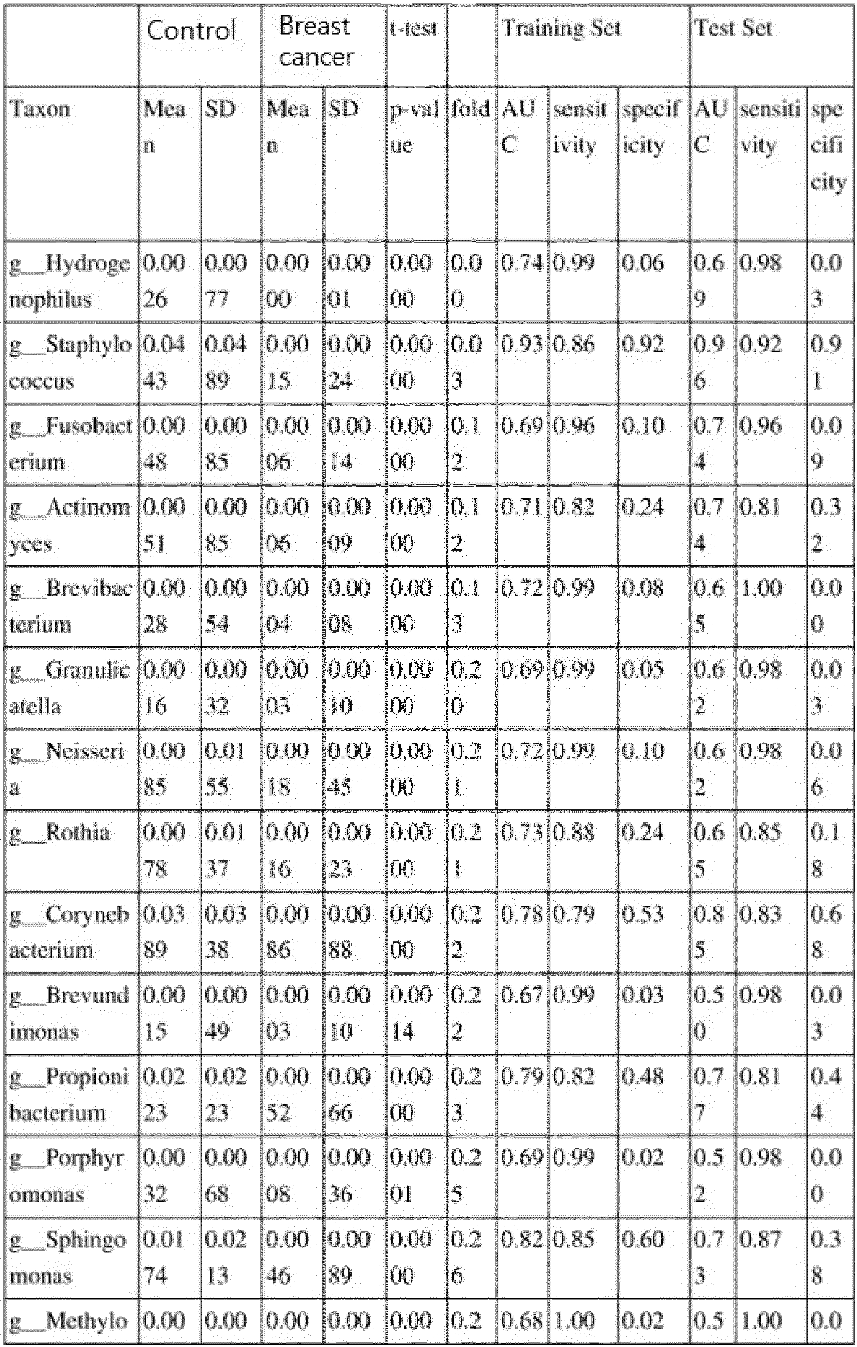

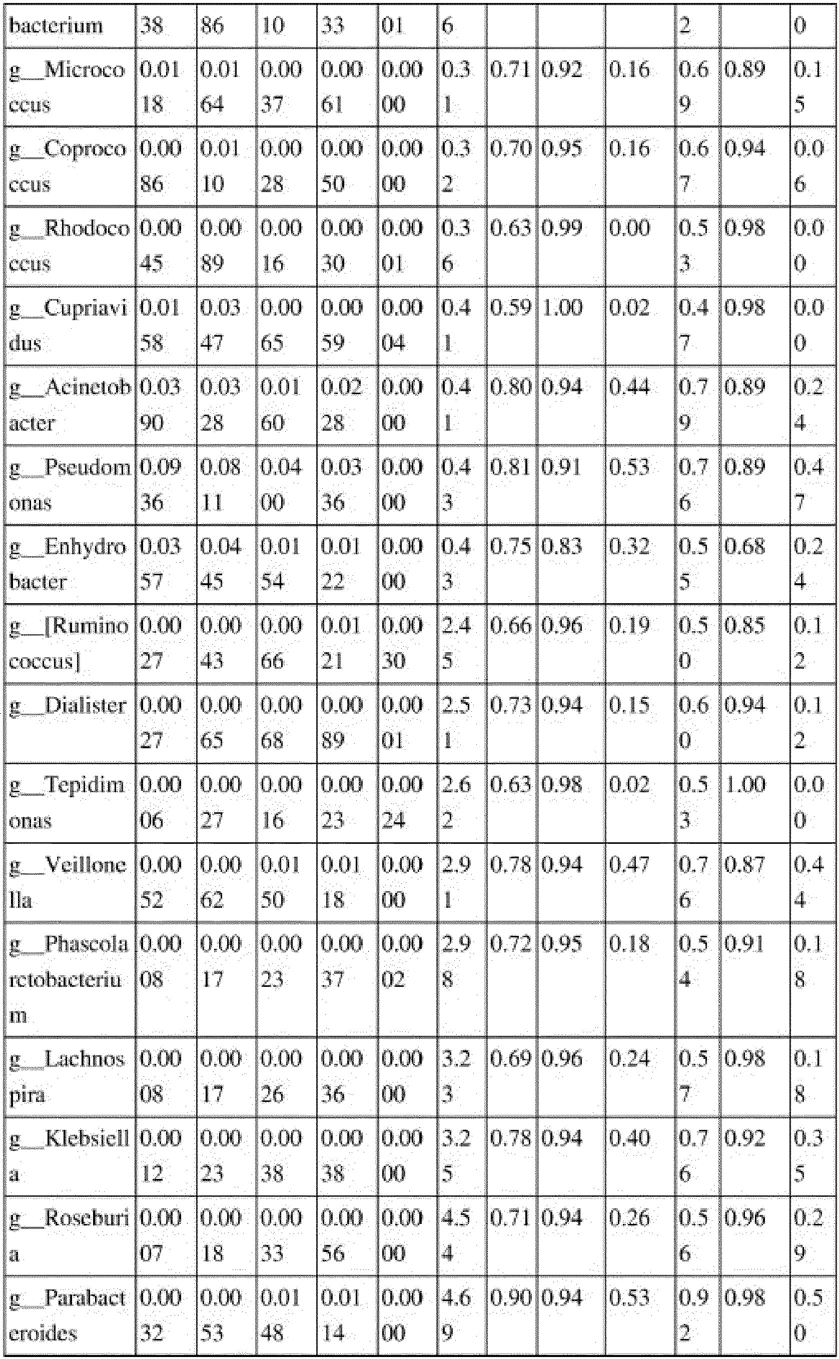

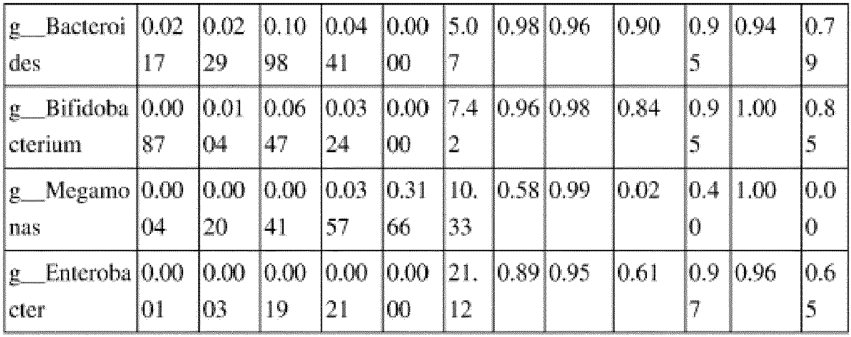

### Example 5. Breast Cancer Diagnostic Model Based on Metagenomic Analysis of Bacteria-Derived EVs Isolated from Urine

EVs were isolated from urine samples of 127 breast cancer patients and 220 normal individuals, the two groups matched in age and gender, and then metagenomic sequencing was performed thereon using the method of Example 3. For the development of a diagnostic model, first, a strain exhibiting a p value of less than 0.05 between two groups in a t-test and a difference of two-fold or more between two groups was selected, and then an AUC, sensitivity, and specificity, which are diagnostic performance indexes, were calculated by logistic regression analysis.

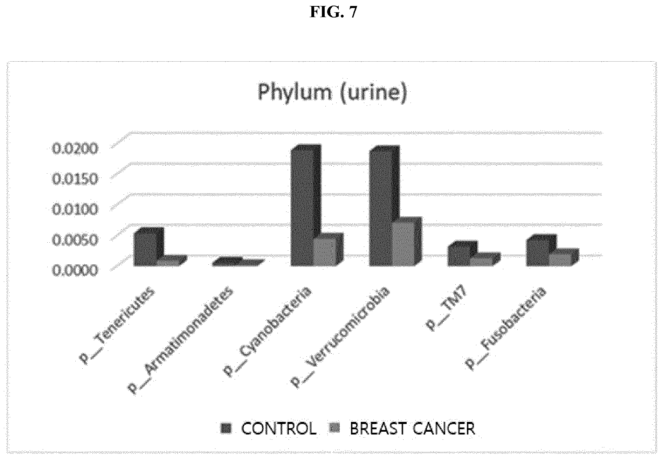

As a result of analyzing bacteria-derived EVs in urine at a phylum level, a diagnostic model developed using bacteria belonging to the phylum *Tenericutes,* the phylum *Armatimonadetes,* the phylum *Cyanobacteria,* the phylum *Verrucomicrobia,* the phylum TM7, and the phylum *Fusobacteria* as a biomarker exhibited significant diagnostic performance for breast cancer (see Table 7 and FIG. 7).

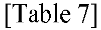

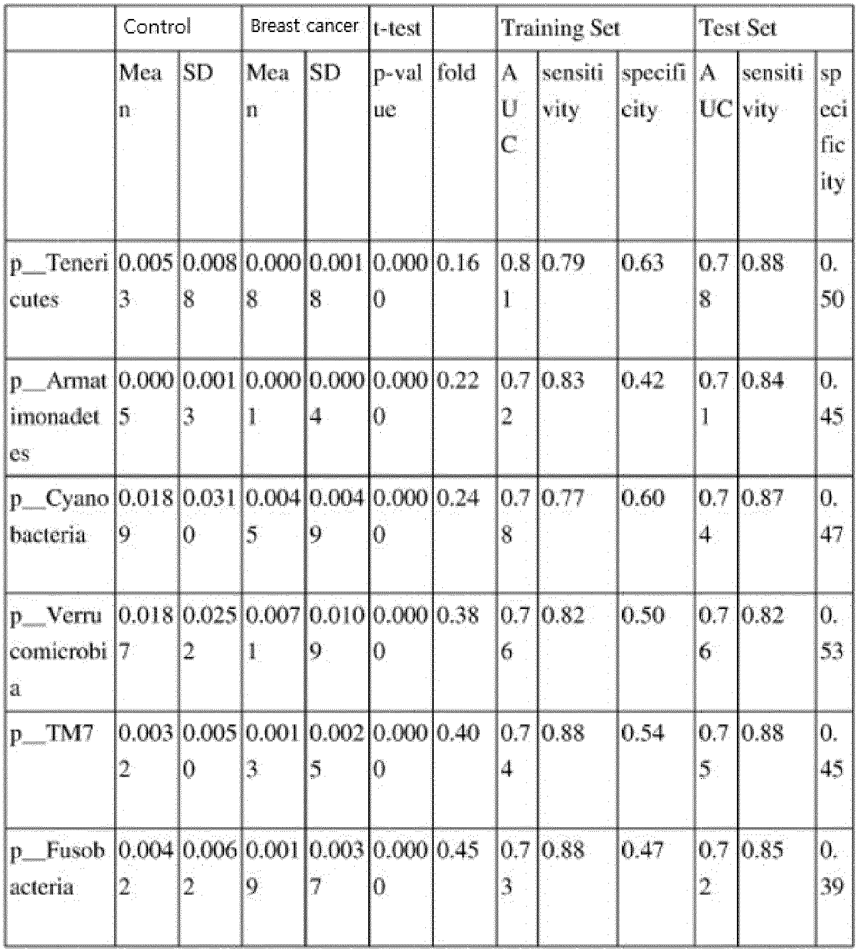

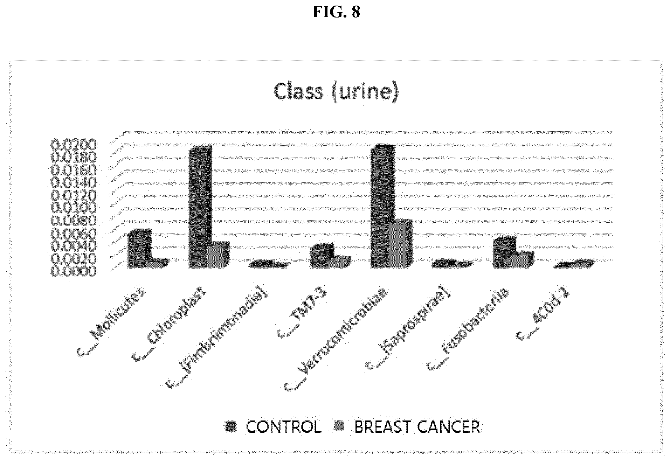

As a result of analyzing bacteria-derived EVs in urine at a class level, a diagnostic model developed using bacteria belonging to the class *Mollicutes,* the class *Chloroplast,* the class *Fimbriimonadia,* the class TM7-3, the class *Verrucomicrobiae,* the class *Saprospirae,* the class *Fusobacteriia,* and the class 4C0d-2 as a biomarker exhibited significant diagnostic performance for breast cancer (see Table 8 and FIG. 8).

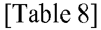

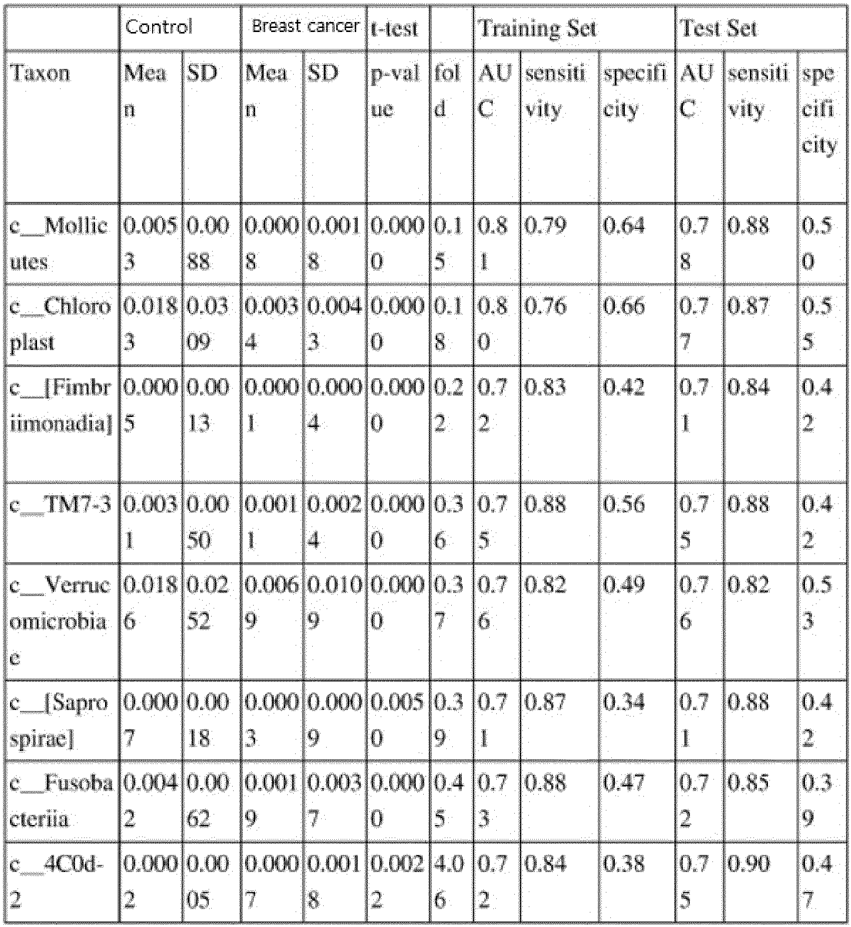

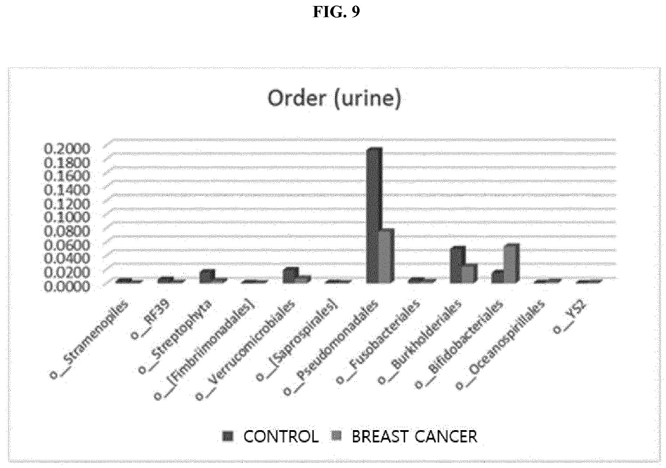

As a result of analyzing bacteria-derived EVs in urine at an order level, a diagnostic model developed using bacteria belonging to the order *Stramenopiles,* the order RF39, the order *Streptophyta,* the order *Fimbriimonadales,* the order *Verrucomicrobiales,* the order *Saprospirales,* the order *Pseudomonadales,* the order *Fusobacteriales,* the order *Burkholderiales,* the order *Bifidobacteriales,* the order *Oceanospirillales,* and the order YS2 as a biomarker exhibited significant diagnostic performance for breast cancer (see Table 9 and FIG. 9).

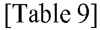

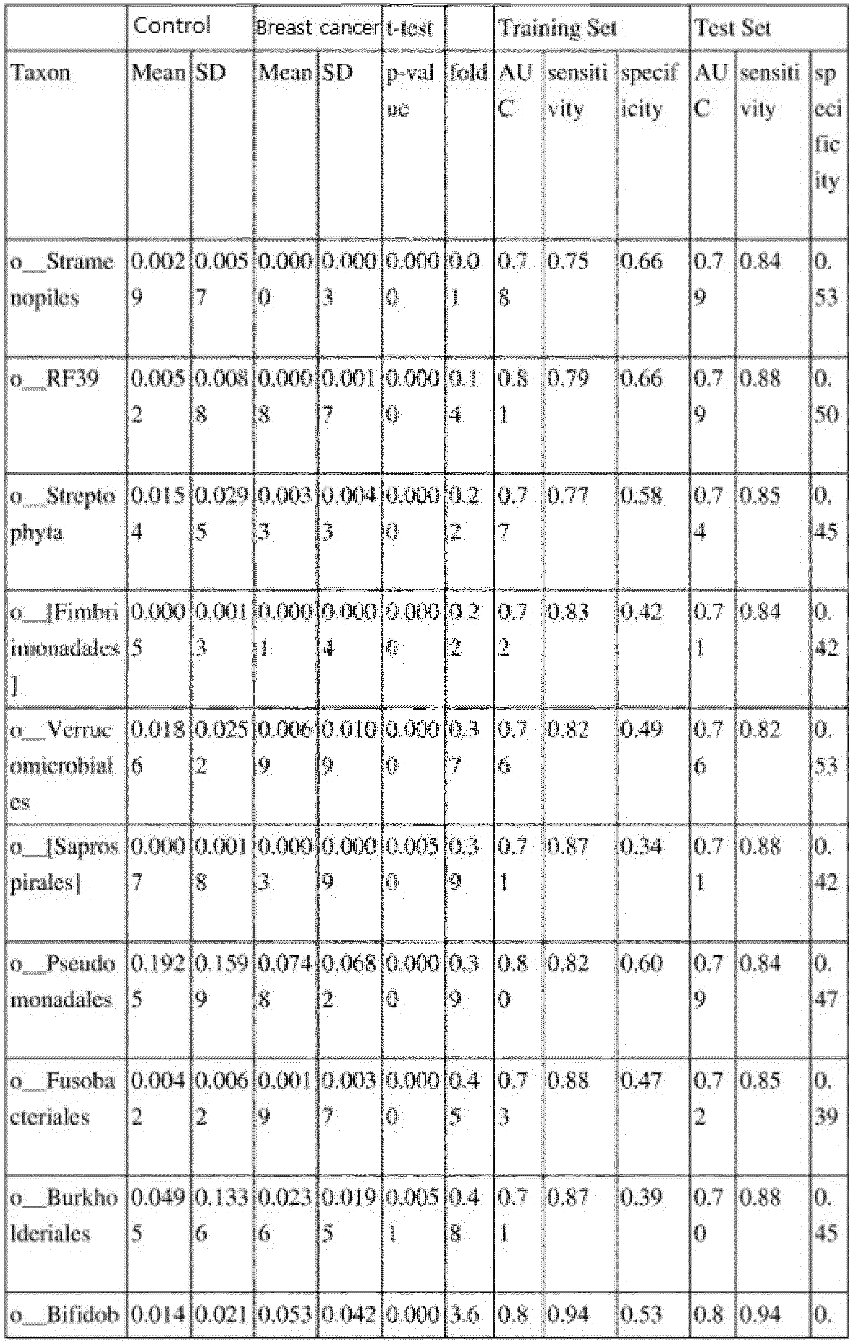

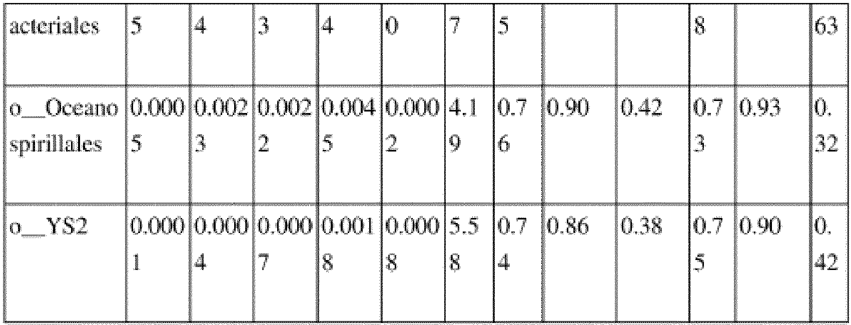

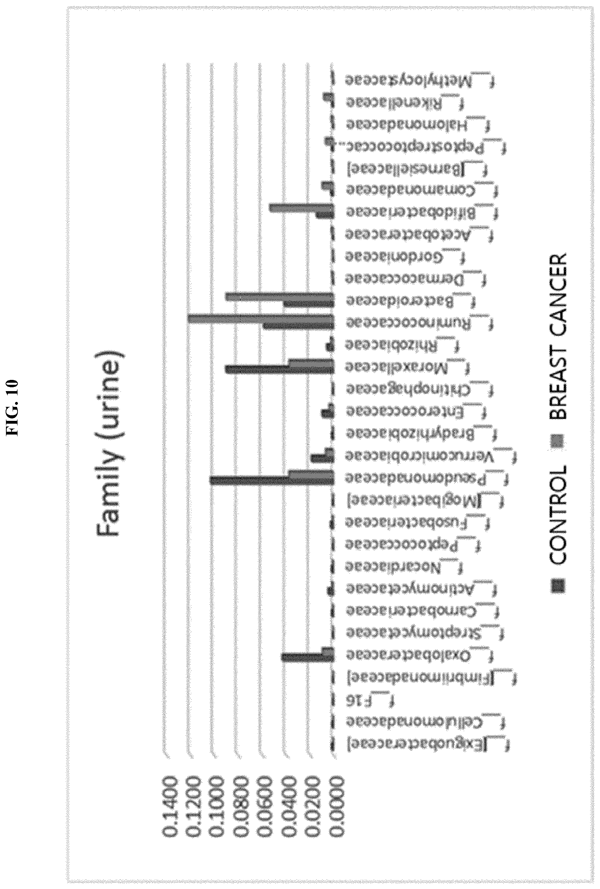

As a result of analyzing bacteria-derived EVs in urine at a family level, a diagnostic model developed using bacteria belonging to the family *Exiguobacteraceae,* the family *Cellulomonadaceae,* the family F16, the family *Fimbriimonadaceae,* the family *Oxalobacteraceae,* the family *Streptomycetaceae,* the family *Carnobacteriaceae,* the family *Actinomycetaceae,* the family *Nocardiaceae,* the family *Peptococcaceae,* the family *Fusobacteriaceae,* the family *Mogibacteriaceae,* the family *Pseudomonadaceae,* the family *Verrucomicrobiaceae,* the family *Bradyrhizobiaceae,* the family *Enterococcaceae,* the family *Chitinophagaceae,* the family *Moraxellaceae,* the family *Rhizobiaceae,* the family *Ruminococcaceae,* the family *Bacteroidaceae,* the family *Dermacoccaceae,* the family *Gordoniaceae,* the family *Acetobacteraceae,* the family *Bifidobacteriaceae,* the family *Comamonadaceae,* the family *Barnesiellaceae,* the family *Peptostreptococcaceae,* the family *Halomonadaceae,* the family *Rikenellaceae,* and the family *Methylocystaceae* as a biomarker exhibited significant diagnostic performance for breast cancer (see Table 10 and FIG. 10).

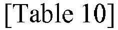

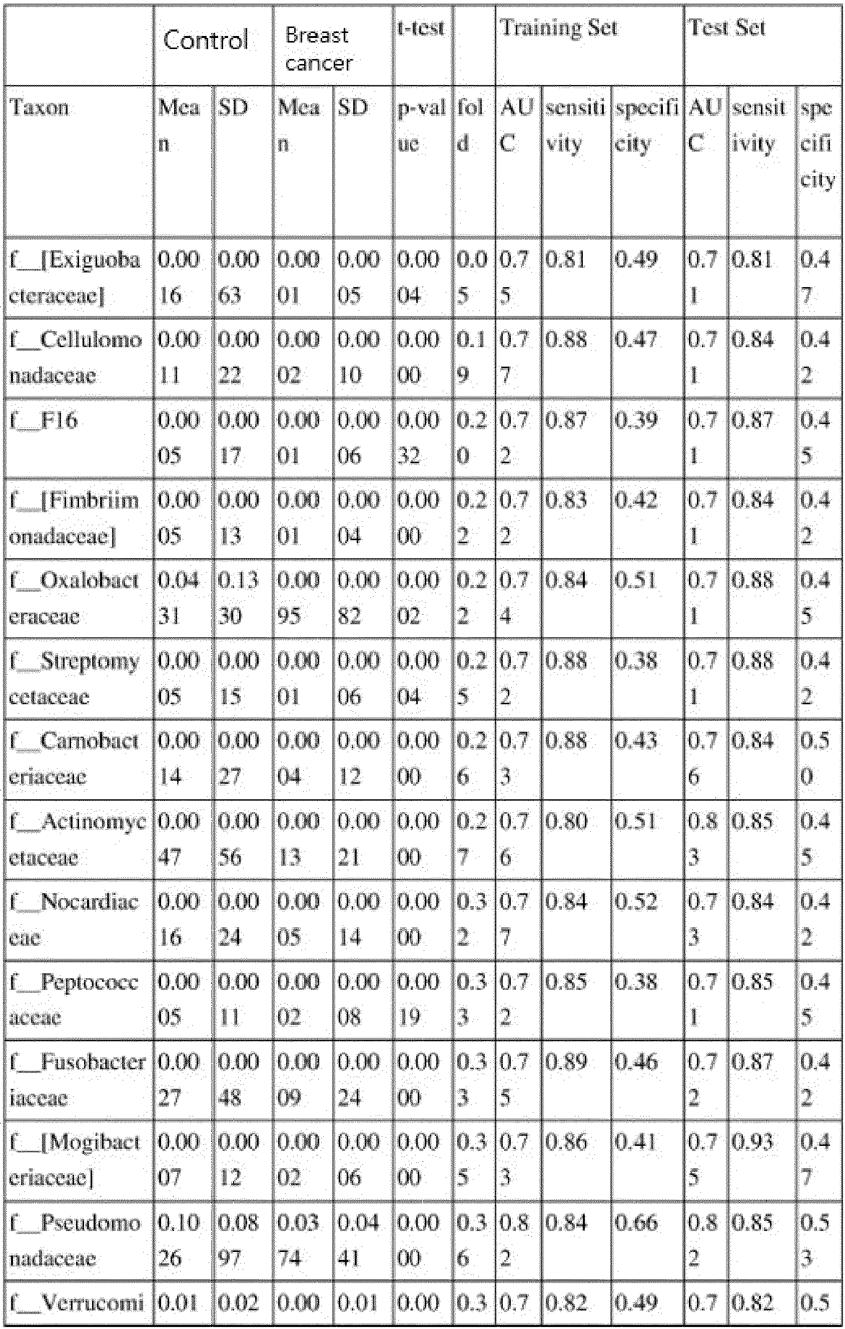

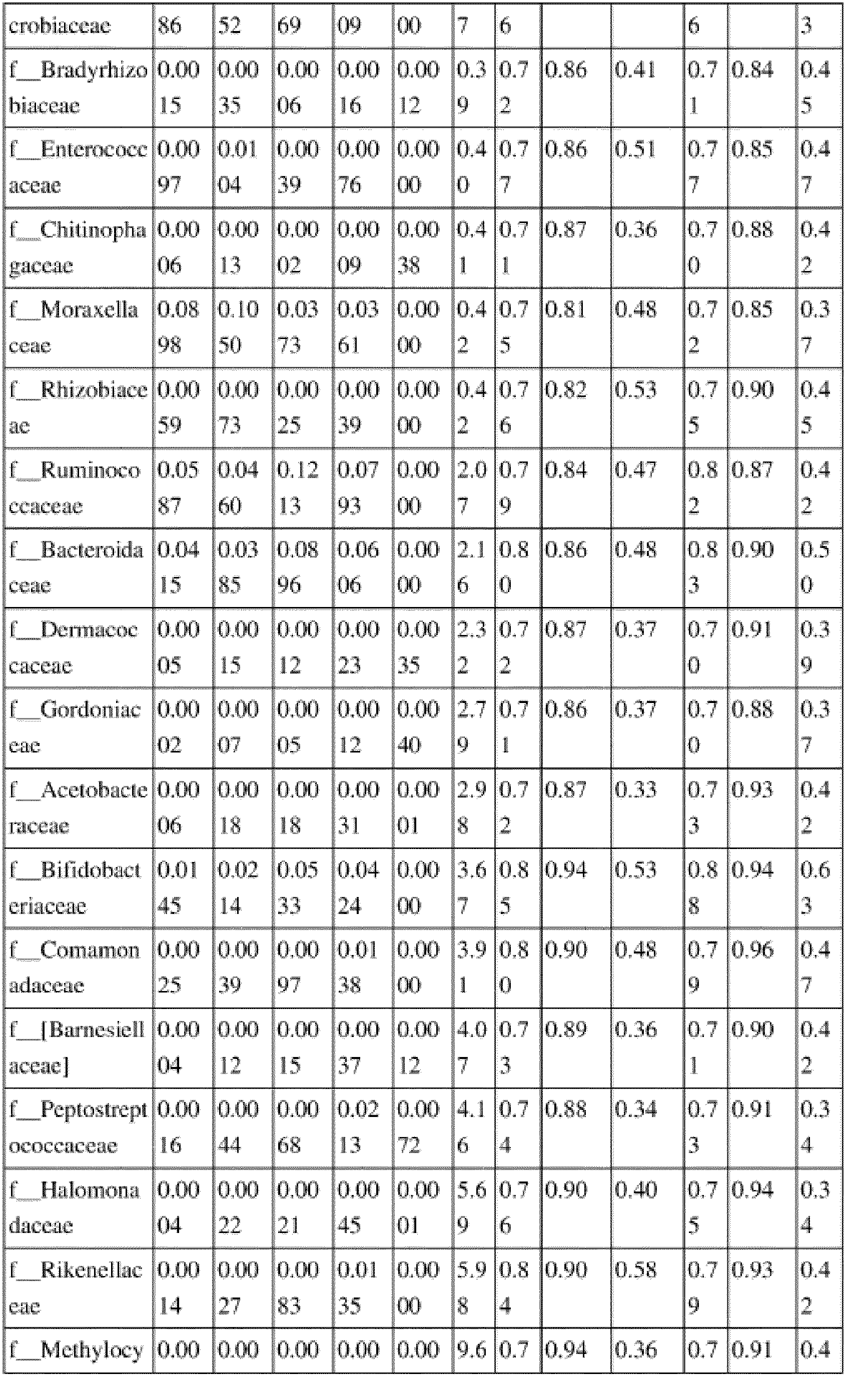

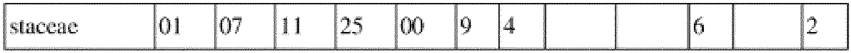

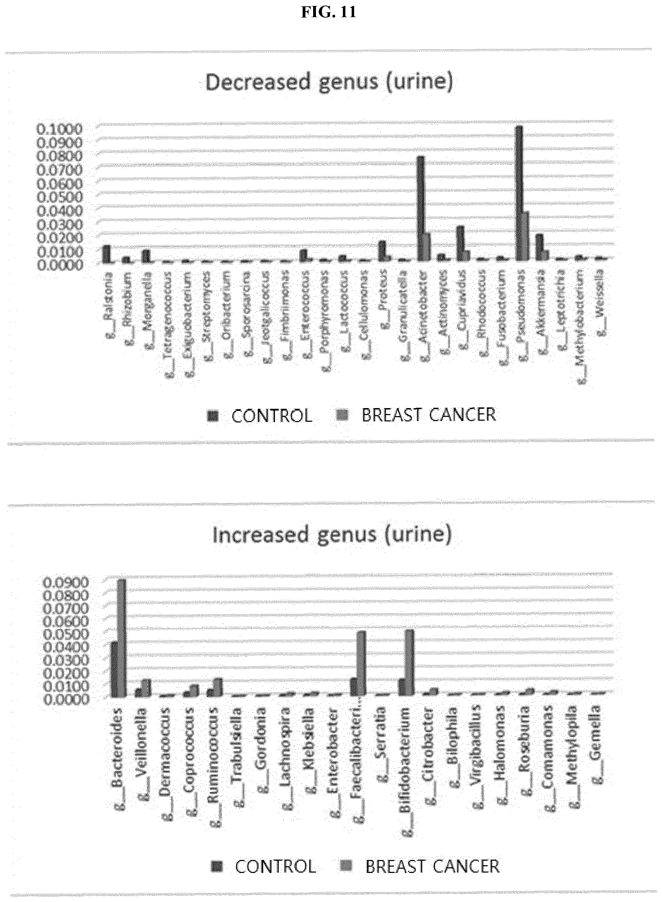

As a result of analyzing bacteria-derived EVs in urine at a genus level, a diagnostic model developed using bacteria belonging to the genus *Ralstonia,* the genus *Rhizobium,* the genus *Morganella,* the genus *Tetragenococcus,* the genus *Exiguobacterium,* the genus *Streptomyces,* the genus *Oribacterium,* the genus *Sporosarcina,* the genus *Jeotgalicoccus,* the genus *Fimbriimonas,* the genus *Enterococcus,* the genus *Porphyromonas,* the genus *Lactococcus,* the genus *Cellulomonas,* the genus *Proteus,* the genus *Granulicatella,* the genus *Acinetobacter,* the genus *Actinomyces,* the genus *Cupriavidus,* the genus *Rhodococcus,* the genus *Fusobacterium,* the genus *Pseudomonas,* the genus *Akkermansia,* the genus *Leptotrichia,* the genus *Methylobacterium,* the genus *Weissella,* the genus *Bacteroides,* the genus *Veillonella,* the genus *Dermacoccus,* the genus *Coprococcus,* the genus *Ruminococcus,* the genus *Trabulsiella,* the genus *Gordonia,* the genus *Lachnospira,* the genus *Klebsiella,* the genus *Enterobacter,* the genus *Faecalibacterium,* the genus *Serratia,* the genus *Bifidobacterium,* the genus *Citrobacter,* the genus *Bilophila,* the genus *Virgibacillus,* the genus *Halomonas,* the genus *Roseburia,* the genus *Comamonas,* the genus *Methylopila,* and the genus *Gemella* as a biomarker exhibited significant diagnostic performance for breast cancer (see Table 11 and FIG. 11).

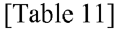

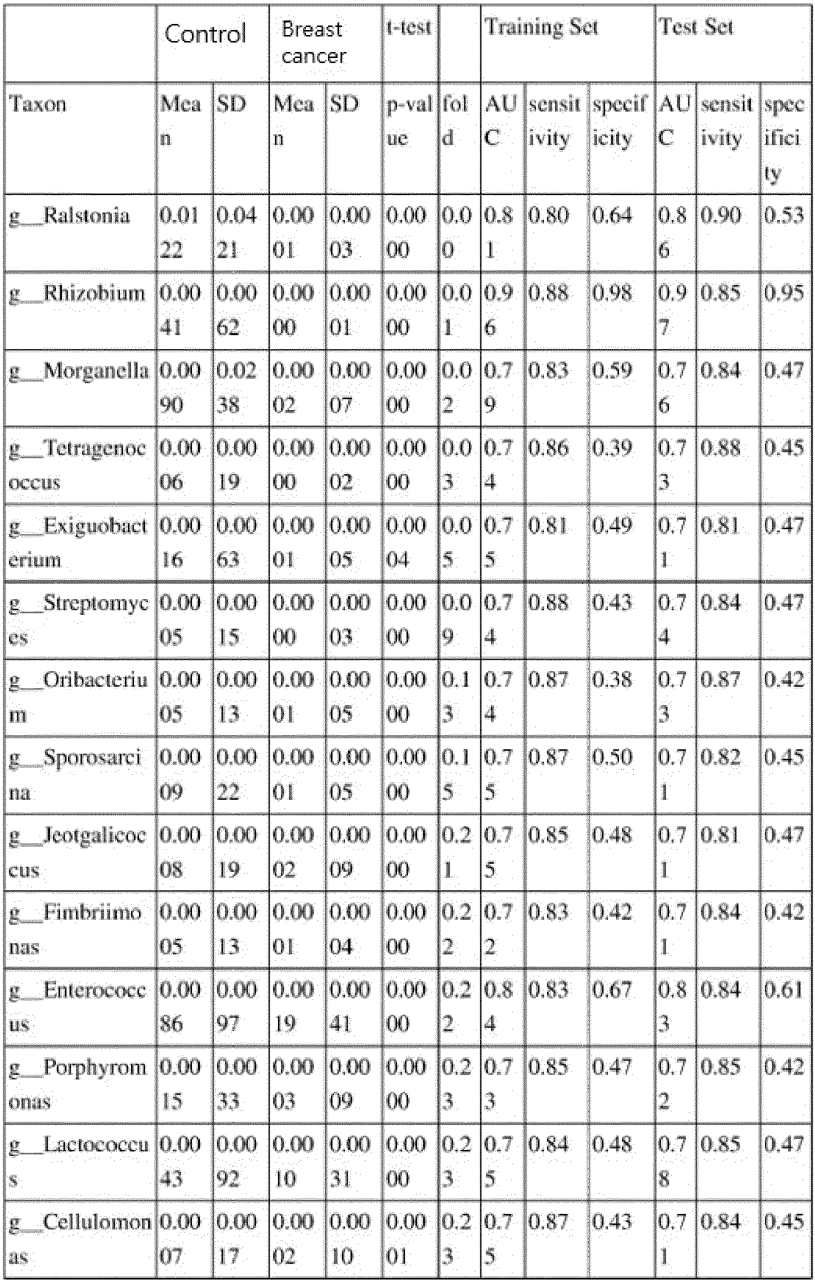

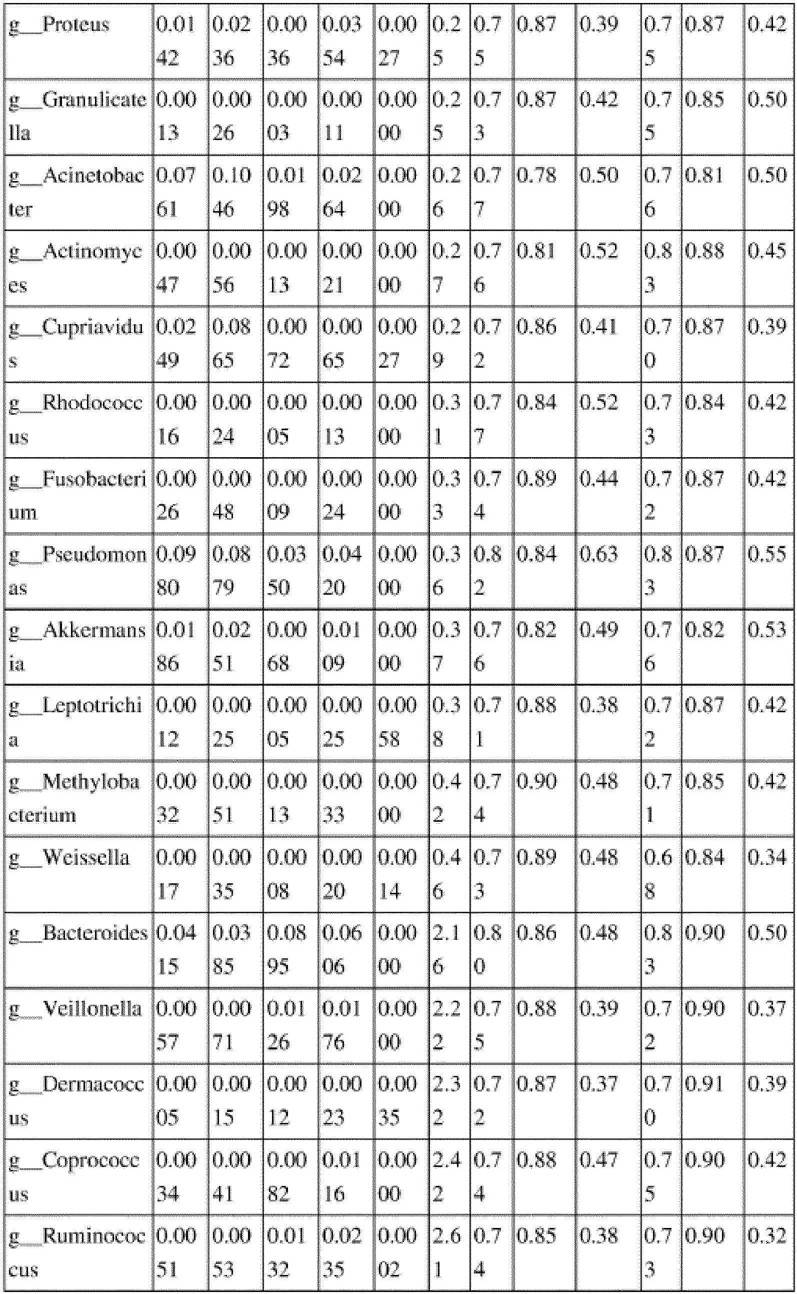

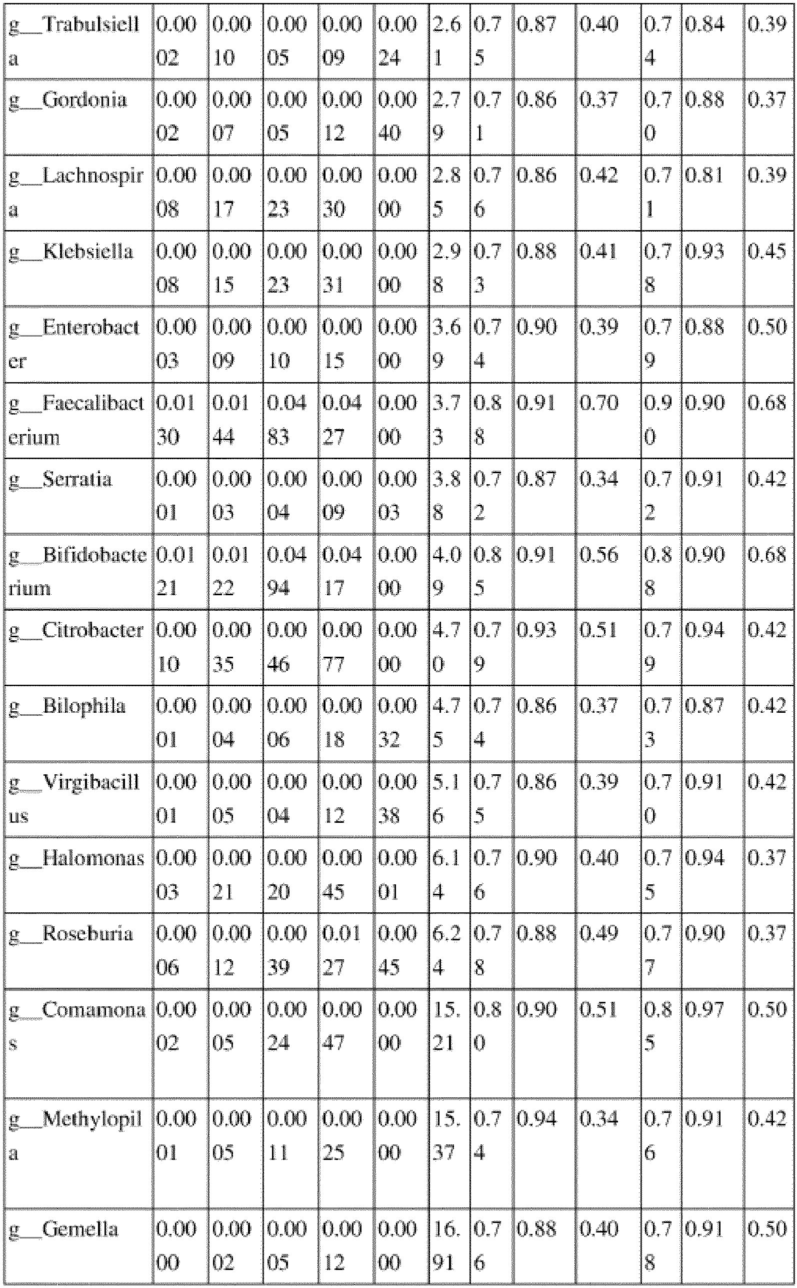

The above description of the present invention is provided only for illustrative purposes. Thus, the embodiments described herein should be considered in an illustrative sense only and not for the purpose of limitation.

### [Industrial Applicability]

According to the present invention, a risk of developing breast cancer may be predicted through metagenomic analysis of bacteria-derived extracellular vesicles by using a human body-derived sample, and thus the onset of breast cancer may be delayed or breast cancer may be prevented through appropriate management by early diagnosis and prediction of a risk group for breast cancer, and, even after breast cancer occurs, early diagnosis for breast cancer may be implemented, thereby lowering the incidence rate of breast cancer and increasing therapeutic effects. In addition, patients diagnosed with breast cancer are able to avoid exposure to causative factors predicted by metagenomic analysis, whereby the progression of cancer may be ameliorated, or recurrence of breast cancer may be prevented.
## Claims

1. A method of providing information for breast cancer diagnosis, the method comprising:
(a) extracting DNA from extracellular vesicles isolated from a subject sample;
(b) performing polymerase chain reaction (PCR) on the extracted DNA using a pair of primers having SEQ ID NO: 1 and SEQ ID NO: 2; and
(c) comparing an increase or decrease in content of bacteria-derived extracellular vesicles of the subject sample with that of a normal individual-derived sample through sequencing of a product of the PCR,
wherein the comparing comprises comparing between blood samples an increase or decrease in content of extracellular vesicles derived from
(i) one or more bacteria selected from the group consisting of the phylum *Fusobacteria* and the phylum *Bacteroidetes;*
(ii) one or more bacteria selected from the group consisting of the class *Fusobacteriia,* the class *Alphaproteobacteria,* the class *Chloroplast,* the class TM7-3, and the class *Bacteroidia;*
(iii) one or more bacteria selected from the group consisting of the order *Fusobacteriales,* the order *Sphingomonadales,* the order *Neisseriales,* the order *Rhizobiales,* the order *Rhodospirillales,* the order *Actinomycetales,* the order *Bacillales,* the order *Streptophyta,* the order *Caulobacterales,* the order *Pseudomonadales,* the order *Bacteroidales,* the order *Enterobacteriales,* and the order *Bifidobacteriales;*
(iv) one or more bacteria selected from the group consisting of the family *Staphylococcaceae,* the family *Fusobacteriaceae,* the family *Actinomycetaceae,* the family *Brevibacteriaceae,* the family *Peptostreptococcaceae,* the family *Rhizobiaceae,* the family *Corynebacteriaceae,* the family *Methylobacteriaceae,* the family *Propionibacteriaceae,* the family *Sphingomonadaceae,* the family *Neisseriaceae,* the family *Flavobacteriaceae,* the family *Intrasporangiaceae,* the family *Nocardiaceae,* the family *Caulobacteraceae,* the family *Micrococcaceae,* the family *Oxalobacteraceae,* the family *Moraxellaceae,* the family *Pseudomonadaceae,* the family *Porphyromonadaceae,* the family *Clostridiaceae,* the family *Rikenellaceae,* the family *Veillonellaceae,* the family *Enterobacteriaceae,* the family S24-7, the family *Bacteroidaceae,* and the family *Bifidobacteriaceae;* or
(v) one or more bacteria selected from the group consisting of the genus *Hydrogenophilus,* the genus *Staphylococcus,* the genus *Fusobacterium,* the genus *Actinomyces,* the genus *Brevibacterium,* the genus *Granulicatella,* the genus *Neisseria,* the genus *Rothia,* the genus *Corynebacterium,* the genus *Brevundimonas,* the genus *Propionibacterium,* the genus *Porphyromonas,* the genus *Sphingomonas,* the genus *Methylobacterium,* the genus *Micrococcus,* the genus *Coprococcus,* the genus *Rhodococcus,* the genus *Cupriavidus,* the genus *Acinetobacter,* the genus *Pseudomonas,* the genus *Enhydrobacter,* the genus *Ruminococcus,* the genus *Dialister,* the genus *Tepidimonas,* the genus *Veillonella,* the genus *Phascolarctobacterium,* the genus *Lachnospira,* the genus *Klebsiella,* the genus *Roseburia,* the genus *Parabacteroides,* the genus *Bacteroides,* the genus *Bifidobacterium,* the genus *Megamonas,* and the genus *Enterobacter;*
or wherein the comparing comprises comparing between urine samples an increase or decrease in content of extracellular vesicles derived from
(vi) one or more bacteria selected from the group consisting of the phylum *Tenericutes,* the phylum *Armatimonadetes,* the phylum *Cyanobacteria,* the phylum *Verrucomicrobia,* the phylum TM7, and the phylum *Fusobacteria;*
(vii) one or more bacteria selected from the group consisting of the class *Mollicutes,* the class *Chloroplast,* the class *Fimbriimonadia,* the class TM7-3, the class *Verrucomicrobiae,* the class *Saprospirae,* the class *Fusobacteriia,* and the class 4C0d-2;
(viii) one or more bacteria selected from the group consisting of the order *Stramenopiles,* the order RF39, the order *Streptophyta,* the order *Fimbriimonadales,* the order *Verrucomicrobiales,* the order *Saprospirales,* the order *Pseudomonadales,* the order *Fusobacteriales,* the order *Burkholderiales,* the order *Bifidobacteriales,* the order *Oceanospirillales,* and the order YS2;
(ix) one or more bacteria selected from the group consisting of the family *Exiguobacteraceae,* the family *Cellulomonadaceae,* the family F16, the family *Fimbriimonadaceae,* the family *Oxalobacteraceae,* the family *Streptomycetaceae,* the family *Carnobacteriaceae,* the family *Actinomycetaceae,* the family *Nocardiaceae,* the family *Peptococcaceae,* the family *Fusobacteriaceae,* the family *Mogibacteriaceae,* the family *Pseudomonadaceae,* the family *Verrucomicrobiaceae,* the family *Bradyrhizobiaceae,* the family *Enterococcaceae,* the family *Chitinophagaceae,* the family *Moraxellaceae,* the family *Rhizobiaceae,* the family *Ruminococcaceae,* the family *Bacteroidaceae,* the family *Dermacoccaceae,* the family *Gordoniaceae,* the family *Acetobacteraceae,* the family *Bifidobacteriaceae,* the family *Comamonadaceae,* the family *Barnesiellaceae,* the family *Peptostreptococcaceae,* the family *Halomonadaceae,* the family *Rikenellaceae,* and the family *Methylocystaceae;* or
(x) one or more bacteria selected from the group consisting of the genus *Ralstonia,* the genus *Rhizobium,* the genus *Morganella,* the genus *Tetragenococcus,* the genus *Exiguobacterium,* the genus *Streptomyces,* the genus *Oribacterium,* the genus *Sporosarcina,* the genus *Jeotgalicoccus,* the genus *Fimbriimonas,* the genus *Enterococcus,* the genus *Porphyromonas,* the genus *Lactococcus,* the genus *Cellulomonas,* the genus *Proteus,* the genus *Granulicatella,* the genus *Acinetobacter,* the genus *Actinomyces,* the genus *Cupriavidus,* the genus *Rhodococcus,* the genus *Fusobacterium,* the genus *Pseudomonas,* the genus *Akkermansia,* the genus *Leptotrichia,* the genus *Methylobacterium,* the genus *Weissella,* the genus *Bacteroides,* the genus *Veillonella,* the genus *Dermacoccus,* the genus *Coprococcus,* the genus *Ruminococcus,* the genus *Trabulsiella,* the genus *Gordonia,* the genus *Lachnospira,* the genus *Klebsiella,* the genus *Enterobacter,* the genus *Faecalibacterium,* the genus *Serratia,* the genus *Bifidobacterium,* the genus *Citrobacter,* the genus *Bilophila,* the genus *Virgibacillus,* the genus *Halomonas,* the genus *Roseburia,* the genus *Comamonas,* the genus *Methylopila,* and the genus *Gemella.*

2. The method of claim 1, wherein the blood is whole blood, serum, or plasma.

3. A method of diagnosing breast cancer, the method comprising:
(a) extracting DNA from extracellular vesicles isolated from a subject sample;
(b) performing polymerase chain reaction (PCR) on the extracted DNA using a pair of primers having SEQ ID NO: 1 and SEQ ID NO: 2; and
(c) comparing an increase or decrease in content of bacteria-derived extracellular vesicles of the subject sample with that of a normal individual-derived sample through sequencing of a product of the PCR; and
(d) diagnosing breast cancer by analyzing the increase or decrease in content of bacteria-derived extracellular vesicles,
wherein the comparing comprises comparing between blood samples an increase or decrease in content of extracellular vesicles derived from
(i) one or more bacteria selected from the group consisting of the phylum *Fusobacteria* and the phylum *Bacteroidetes;*
(ii) one or more bacteria selected from the group consisting of the class *Fusobacteriia,* the class *Alphaproteobacteria,* the class *Chloroplast,* the class TM7-3, and the class *Bacteroidia;*
(iii) one or more bacteria selected from the group consisting of the order *Fusobacteriales,* the order *Sphingomonadales,* the order *Neisseriales,* the order *Rhizobiales,* the order *Rhodospirillales,* the order *Actinomycetales,* the order *Bacillales,* the order *Streptophyta,* the order *Caulobacterales,* the order *Pseudomonadales,* the order *Bacteroidales,* the order *Enterobacteriales,* and the order *Bifidobacteriales;*
(iv) one or more bacteria selected from the group consisting of the family *Staphylococcaceae,* the family *Fusobacteriaceae,* the family *Actinomycetaceae,* the family *Brevibacteriaceae,* the family *Peptostreptococcaceae,* the family *Rhizobiaceae,* the family *Corynebacteriaceae,* the family *Methylobacteriaceae,* the family *Propionibacteriaceae,* the family *Sphingomonadaceae,* the family *Neisseriaceae,* the family *Flavobacteriaceae,* the family *Intrasporangiaceae,* the family *Nocardiaceae,* the family *Caulobacteraceae,* the family *Micrococcaceae,* the family *Oxalobacteraceae,* the family *Moraxellaceae,* the family *Pseudomonadaceae,* the family *Porphyromonadaceae,* the family *Clostridiaceae,* the family *Rikenellaceae,* the family *Veillonellaceae,* the family *Enterobacteriaceae,* the family S24-7, the family *Bacteroidaceae,* and the family *Bifidobacteriaceae;* or
(v) one or more bacteria selected from the group consisting of the genus *Hydrogenophilus,* the genus *Staphylococcus,* the genus *Fusobacterium,* the genus *Actinomyces,* the genus *Brevibacterium,* the genus *Granulicatella,* the genus *Neisseria,* the genus *Rothia,* the genus *Corynebacterium,* the genus *Brevundimonas,* the genus *Propionibacterium,* the genus *Porphyromonas,* the genus *Sphingomonas,* the genus *Methylobacterium,* the genus *Micrococcus,* the genus *Coprococcus,* the genus *Rhodococcus,* the genus *Cupriavidus,* the genus *Acinetobacter,* the genus *Pseudomonas,* the genus *Enhydrobacter,* the genus *Ruminococcus,* the genus *Dialister,* the genus *Tepidimonas,* the genus *Veillonella,* the genus *Phascolarctobacterium,* the genus *Lachnospira,* the genus *Klebsiella,* the genus *Roseburia,* the genus *Parabacteroides,* the genus *Bacteroides,* the genus *Bifidobacterium,* the genus *Megamonas,* and the genus *Enterobacter;*
or wherein the comparing comprises comparing between urine samples an increase or decrease in content of extracellular vesicles derived from
(vi) one or more bacteria selected from the group consisting of the phylum *Tenericutes,* the phylum *Armatimonadetes,* the phylum *Cyanobacteria,* the phylum *Verrucomicrobia,* the phylum TM7, and the phylum *Fusobacteria;*
(vii) one or more bacteria selected from the group consisting of the class *Mollicutes,* the class *Chloroplast,* the class *Fimbriimonadia,* the class TM7-3, the class *Verrucomicrobiae,* the class *Saprospirae,* the class *Fusobacteriia,* and the class 4C0d-2;
(viii) one or more bacteria selected from the group consisting of the order *Stramenopiles,* the order RF39, the order *Streptophyta,* the order *Fimbriimonadales,* the order *Verrucomicrobiales,* the order *Saprospirales,* the order *Pseudomonadales,* the order *Fusobacteriales,* the order *Burkholderiales,* the order *Bifidobacteriales,* the order *Oceanospirillales,* and the order YS2;
(ix) one or more bacteria selected from the group consisting of the family *Exiguobacteraceae,* the family *Cellulomonadaceae,* the family F16, the family *Fimbriimonadaceae,* the family *Oxalobacteraceae,* the family *Streptomycetaceae,* the family *Carnobacteriaceae,* the family *Actinomycetaceae,* the family *Nocardiaceae,* the family *Peptococcaceae,* the family *Fusobacteriaceae,* the family *Mogibacteriaceae,* the family *Pseudomonadaceae,* the family *Verrucomicrobiaceae,* the family *Bradyrhizobiaceae,* the family *Enterococcaceae,* the family *Chitinophagaceae,* the family *Moraxellaceae,* the family *Rhizobiaceae,* the family *Ruminococcaceae,* the family *Bacteroidaceae,* the family *Dermacoccaceae,* the family *Gordoniaceae,* the family *Acetobacteraceae,* the family *Bifidobacteriaceae,* the family *Comamonadaceae,* the family *Barnesiellaceae,* the family *Peptostreptococcaceae,* the family *Halomonadaceae,* the family *Rikenellaceae,* and the family *Methylocystaceae;* or
(x) one or more bacteria selected from the group consisting of the genus *Ralstonia,* the genus *Rhizobium,* the genus *Morganella,* the genus *Tetragenococcus,* the genus *Exiguobacterium,* the genus *Streptomyces,* the genus *Oribacterium,* the genus *Sporosarcina,* the genus *Jeotgalicoccus,* the genus *Fimbriimonas,* the genus *Enterococcus,* the genus *Porphyromonas,* the genus *Lactococcus,* the genus *Cellulomonas,* the genus *Proteus,* the genus *Granulicatella,* the genus *Acinetobacter,* the genus *Actinomyces,* the genus *Cupriavidus,* the genus *Rhodococcus,* the genus *Fusobacterium,* the genus *Pseudomonas,* the genus *Akkermansia,* the genus *Leptotrichia,* the genus *Methylobacterium,* the genus *Weissella,* the genus *Bacteroides,* the genus *Veillonella,* the genus *Dermacoccus,* the genus *Coprococcus,* the genus *Ruminococcus,* the genus *Trabulsiella,* the genus *Gordonia,* the genus *Lachnospira,* the genus *Klebsiella,* the genus *Enterobacter,* the genus *Faecalibacterium,* the genus *Serratia,* the genus *Bifidobacterium,* the genus *Citrobacter,* the genus *Bilophila,* the genus *Virgibacillus,* the genus *Halomonas,* the genus *Roseburia,* the genus *Comamonas,* the genus *Methylopila,* and the genus *Gemella.*

4. The method of claim 3, wherein the blood is whole blood, serum, or plasma.

## Patentansprüche

1. Verfahren zum Bereitstellen von Informationen über eine Brustkrebsdiagnose, wobei das Verfahren Folgendes umfasst:
(a) Extrahieren von DNA aus extrazellulären Vesikeln, die aus einer Patientenprobe isoliert wurden;
(b) Durchführen einer Polymerase-Kettenreaktion (PCR) an der extrahierten DNA unter Verwendung eines Paars von Primern, welche SEQ ID NO: 1 und SEQ ID NO: 2 aufweisen; und
(c) Vergleichen einer Erhöhung oder Verringerung des Gehalts von bakterienabgeleiteten, extrazellulären Vesikeln der Patientenprobe mit der einer normalen, individuell abgeleiteten Probe durch Sequenzierung eines Produkts der PCR, wobei das Vergleichen das Vergleichen zwischen Blutproben einer Erhöhung oder Verringerung des Gehalts von extrazellulären Vesikeln von Folgendem abgeleitet ist
(i) einem oder mehreren Bakterien, die ausgewählt sind aus der Gruppe bestehend aus dem Phylum Fusobacteria und dem Phylum Bacteroidetes;
(ii) einem oder mehreren Bakterien, die ausgewählt sind aus der Gruppe bestehend aus der Fusobacteriia-Klasse, der Alphaproteobacteria-Klasse, der Chloroplast-Klasse, der TM7- 3-Klasse und der Bacteroidia-Klasse;
(iii) einem oder mehreren Bakterien, die ausgewählt sind aus der Gruppe bestehend aus der Reihe von Fusobacteriales, der Reihe von Sphingomonadales, der Reihe von Neisseriales, der Reihe von Rhizobiales, der Reihe von Rhodospirillales, der Reihe von Actinomycetales, der Reihe von Bacillales, der Reihe von Streptophyta, der Reihe von Caulobacterales, der Reihe von Pseudomonadales, der Reihe von Bacteroidales, der Reihe von Enterobacteriales und der Reihe von Bifidobacteriales;
(iv) einem oder mehreren Bakterien, die ausgewählt sind aus der Gruppe bestehend aus der Staphylococcaceae-Familie, der Fusobacteriaceae-Familie, der Actinomycetaceae-Familie, der Brevibacteriaceae-Familie, der Peptostreptococcaceae-Familie, der Rhizobiaceae-Familie, der Corynebacteriaceae-Familie, der Methylobacteriaceae-Familie, der Propionibacteriaceae-Familie, der Sphingomonadaceae-Familie, der Neisseriaceae-Familie, der Flavobacteriaceae-Familie, der Intrasporangiaceae-Familie, der Nocardiaceae-Familie, der Caulobacteraceae-Familie, der Micrococcaceae-Familie, der Oxalobacteraceae-Familie, der Moraxellaceae-Familie, der Pseudomonadaceae-Familie, der Porphyromonadaceae-Familie, der Clostridiaceae-Familie, der Rikenellaceae-Familie, der Veillonellaceae-Familie, der Enterobacteriaceae-Familie, der S24-7-Familie, der Bacteroidaceae-Familie und der Bifidobacteriaceae-Familie; oder
(v) einem oder mehreren Bakterien, die ausgewählt sind aus der Gruppe bestehend aus der Gattung Hydrogenophilus, der Gattung Staphylococcus, der Gattung Fusobacterium, der Gattung Actinomyces, der Gattung Brevibacterium, der Gattung Granulicatella, der Gattung Neisseria, der Gattung Rothia, der Gattung Corynebacterium, der Gattung Brevundimonas, der Gattung Propionibacterium, der Gattung Porphyromonas, der Gattung Sphingomonas, der Gattung Methylobacterium, der Gattung Micrococcus, der Gattung Coprococcus, der Gattung Rhodococcus, der Gattung Cupriavidus, der Gattung Acinetobacter, der Gattung Pseudomonas, der Gattung Enhydrobacter, der Gattung Ruminococcus, der Gattung Dialister, der Gattung Tepidimonas, der Gattung Veillonella, der Gattung Phascolarctobacterium, der Gattung Lachnospira, der Gattung Klebsiella, der Gattung Roseburia, der Gattung Parabacteroides, der Gattung Bacteroides, der Gattung Bifidobacterium, der Gattung Megamonas und der Gattung Enterobacter;
oder wobei das Vergleichen das Vergleichen zwischen Urinproben einer Erhöhung oder Verringerung des Gehalts von extrazellulären Vesikeln von Folgendem abgeleitet ist
(vi) einem oder mehreren Bakterien, die ausgewählt sind aus der Gruppe bestehend aus dem Phylum Tenericutes, dem Phylum Armatimonadetes, dem Phylum Cyanobacteria, dem Phylum Verrucomicrobia, dem Phylum TM7 und dem Phylum Fusobacteria;
(vii) einem oder mehreren Bakterien, die ausgewählt sind aus der Gruppe bestehend aus der Mollicutes-Klasse, der Chloroplast-Klasse, der Fimbriimonadia-Klasse, der TM7-3-Klasse, der Verrucomicrobiae-Klasse, der Saprospirae-Klasse, der Fusobacteriia-Klasse und der 4C0d-2-Klasse;
(viii) einem oder mehreren Bakterien, die ausgewählt sind aus der Gruppe bestehend aus der Reihe von Stramenopiles, der Reihe von RF39, der Reihe von Streptophyta, der Reihe von Fimbriimonadales, der Reihe von Verrucomicrobiales, der Reihe von Saprospirales, der Reihe von Pseudomonadales, der Reihe von Fusobacteriales, der Reihe von Burkholderiales, der Reihe von Bifidobacteriales, der Reihe von Oceanospirillales, und der Reihe von YS2;
(ix) einem oder mehreren Bakterien, die ausgewählt sind aus der Gruppe bestehend aus der Exiguobacteraceae-Familie, der Cellulomonadaceae-Familie, der F16-Familie, der Fimbriimonadaceae-Familie, der Oxalobacteraceae-Familie, der Streptomycetaceae-Familie, der Carnobacteriaceae-Familie, der Actinomycetaceae-Familie, der Nocardiaceae-Familie, der Peptococcaceae-Familie, der Fusobacteriaceae-Familie, der Mogibacteriaceae-Familie, der Pseudomonadaceae-Familie, der Verrucomicrobiaceae-Familie, der Bradyrhizobiaceae-Familie, der Enterococcaceae-Familie, der Chitinophagaceae-Familie, der Moraxellaceae-Familie, der Rhizobiaceae-Familie, der Ruminococcaceae-Familie, der Bacteroidaceae-Familie, der Dermacoccaceae-Familie, der Gordoniaceae-Familie, der Acetobacteraceae-Familie, der Bifidobacteriaceae-Familie, der Comamonadaceae-Familie, der Barnesiellaceae-Familie, der Peptostreptococcaceae-Familie, der Halomonadaceae-Familie, der Rikenellaceae-Familie und der Methylocystaceae-Familie; oder
(x) einem oder mehreren Bakterien, die ausgewählt sind aus der Gruppe bestehend aus der Gattung Ralstonia, der Gattung Rhizobium, der Gattung Morganella, der Gattung Tetragenococcus, der Gattung Exiguobacterium, der Gattung Streptomyces, der Gattung Oribacterium, der Gattung Sporosarcina, der Gattung Jeotgalicoccus, der Gattung Fimbriimonas, der Gattung Enterococcus, der Gattung Porphyromonas, der Gattung Lactococcus, der Gattung Cellulomonas, der Gattung Proteus, der Gattung Granulicatella, der Gattung Acinetobacter, der Gattung Actinomyces, der Gattung Cupriavidus, der Gattung Rhodococcus, der Gattung Fusobacterium, der Gattung Pseudomonas, der Gattung Akkermansia, der Gattung Leptotrichia, der Gattung Methylobacterium, der Gattung Weissella, der Gattung Bacteroides, der Gattung Veillonella, der Gattung Dermacoccus, der Gattung Coprococcus, der Gattung Ruminococcus, der Gattung Trabulsiella, der Gattung Gordonia, der Gattung Lachnospira, der Gattung Klebsiella, der Gattung Enterobacter, der Gattung Faecalibacterium, der Gattung Serratia, der Gattung Bifidobacterium, der Gattung Citrobacter, der Gattung Bilophila, der Gattung Virgibacillus, der Gattung Halomonas, der Gattung Roseburia, der Gattung Comamonas, der Gattung Methylopila und der Gattung Gemella.

2. Verfahren nach Anspruch 1, wobei das Blut Vollblut, Serum oder Plasma ist.

3. Verfahren zum Diagnostizieren von Brustkrebs, wobei das Verfahren Folgendes umfasst:
(a) Extrahieren von DNA aus extrazellulären Vesikeln, die aus einer Patientenprobe isoliert wurden;
(b) Durchführen einer Polymerase-Kettenreaktion (PCR) an der extrahierten DNA unter Verwendung eines Paars von Primern, welche SEQ ID NO: 1 und SEQ ID NO: 2 aufweisen; und
(c) Vergleichen einer Erhöhung oder Verringerung des Gehalts von bakterienabgeleiteten, extrazellulären Vesikeln der Patientenprobe mit der einer normalen, individuell abgeleiteten Probe durch Sequenzierung eines Produkts der PCR; und
(d) Diagnostizieren von Brustkrebs durch Analysieren der Erhöhung oder Verringerung des Gehalts von bakterienabgeleiteten, extrazellulären Vesikeln, wobei das Vergleichen das Vergleichen zwischen Blutproben einer Erhöhung oder Verringerung des Gehalts von extrazellulären Vesikeln von Folgendem abgeleitet ist
(i) einem oder mehreren Bakterien, die ausgewählt sind aus der Gruppe bestehend aus dem Phylum Fusobacteria und dem Phylum Bacteroidetes;
(ii) einem oder mehreren Bakterien, die ausgewählt sind aus der Gruppe bestehend aus der Fusobacteriia-Klasse, der Alphaproteobacteria-Klasse, der Chloroplast-Klasse, der TM7- 3-Klasse und der Bacteroidia-Klasse;
(iii) einem oder mehreren Bakterien, die ausgewählt sind aus der Gruppe bestehend aus der Reihe von Fusobacteriales, der Reihe von Sphingomonadales, der Reihe von Neisseriales, der Reihe von Rhizobiales, der Reihe von Rhodospirillales, der Reihe von Actinomycetales, der Reihe von Bacillales, der Reihe von Streptophyta, der Reihe von Caulobacterales, der Reihe von Pseudomonadales, der Reihe von Bacteroidales, der Reihe von Enterobacteriales und der Reihe von Bifidobacteriales;
(iv) einem oder mehreren Bakterien, die ausgewählt sind aus der Gruppe bestehend aus der Staphylococcaceae-Familie, der Fusobacteriaceae-Familie, der Actinomycetaceae-Familie, der Brevibacteriaceae-Familie, der Peptostreptococcaceae-Familie, der Rhizobiaceae-Familie, der Corynebacteriaceae-Familie, der Methylobacteriaceae-Familie, der Propionibacteriaceae-Familie, der Sphingomonadaceae-Familie, der Neisseriaceae-Familie, der Flavobacteriaceae-Familie, der Intrasporangiaceae-Familie, der Nocardiaceae-Familie, der Caulobacteraceae-Familie, der Micrococcaceae-Familie, der Oxalobacteraceae-Familie, der Moraxellaceae-Familie, der Pseudomonadaceae-Familie, der Porphyromonadaceae-Familie, der Clostridiaceae-Familie, der Rikenellaceae-Familie, der Veillonellaceae-Familie, der Enterobacteriaceae-Familie, der S24-7-Familie, der Bacteroidaceae-Familie und der Bifidobacteriaceae-Familie; oder
(v) einem oder mehreren Bakterien, die ausgewählt sind aus der Gruppe bestehend aus der Gattung Hydrogenophilus, der Gattung Staphylococcus, der Gattung Fusobacterium, der Gattung Actinomyces, der Gattung Brevibacterium, der Gattung Granulicatella, der Gattung Neisseria, der Gattung Rothia, der Gattung Corynebacterium, der Gattung Brevundimonas, der Gattung Propionibacterium, der Gattung Porphyromonas, der Gattung Sphingomonas, der Gattung Methylobacterium, der Gattung Micrococcus, der Gattung Coprococcus, der Gattung Rhodococcus, der Gattung Cupriavidus, der Gattung Acinetobacter, der Gattung Pseudomonas, der Gattung Enhydrobacter, der Gattung Ruminococcus, der Gattung Dialister, der Gattung Tepidimonas, der Gattung Veillonella, der Gattung Phascolarctobacterium, der Gattung Lachnospira, der Gattung Klebsiella, der Gattung Roseburia, der Gattung Parabacteroides, der Gattung Bacteroides, der Gattung Bifidobacterium, der Gattung Megamonas und der Gattung Enterobacter;
oder wobei das Vergleichen das Vergleichen zwischen Urinproben einer Erhöhung oder Verringerung des Gehalts von extrazellulären Vesikeln von Folgendem abgeleitet ist
(vi) einem oder mehreren Bakterien, die ausgewählt sind aus der Gruppe bestehend aus dem Phylum Tenericutes, dem Phylum Armatimonadetes, dem Phylum Cyanobacteria, dem Phylum Verrucomicrobia, dem Phylum TM7 und dem Phylum Fusobacteria;
(vii) einem oder mehreren Bakterien, die ausgewählt sind aus der Gruppe bestehend aus der Mollicutes-Klasse, der Chloroplast-Klasse, der Fimbriimonadia-Klasse, der TM7-3-Klasse, der Verrucomicrobiae-Klasse, der Saprospirae-Klasse, der Fusobacteriia-Klasse und der 4COd-2-Klasse;
(viii) einem oder mehreren Bakterien, die ausgewählt sind aus der Gruppe bestehend aus der Reihe von Stramenopiles, der Reihe von RF39, der Reihe von Streptophyta, der Reihe von Fimbriimonadales, der Reihe von Verrucomicrobiales, der Reihe von Saprospirales, der Reihe von Pseudomonadales, der Reihe von Fusobacteriales, der Reihe von Burkholderiales, der Reihe von Bifidobacteriales, der Reihe von Oceanospirillales, und der Reihe von YS2;
(ix) einem oder mehreren Bakterien, die ausgewählt sind aus der Gruppe bestehend aus der Exiguobacteraceae-Familie, der Cellulomonadaceae-Familie, der F16-Familie, der Fimbriimonadaceae-Familie, der Oxalobacteraceae-Familie, der Streptomycetaceae-Familie, der Carnobacteriaceae-Familie, der Actinomycetaceae-Familie, der Nocardiaceae-Familie, der Peptococcaceae-Familie, der Fusobacteriaceae-Familie, der Mogibacteriaceae-Familie, der Pseudomonadaceae-Familie, der Verrucomicrobiaceae-Familie, der Bradyrhizobiaceae-Familie, der Enterococcaceae-Familie, der Chitinophagaceae-Familie, der Moraxellaceae-Familie, der Rhizobiaceae-Familie, der Ruminococcaceae-Familie, der Bacteroidaceae-Familie, der Dermacoccaceae-Familie, der Gordoniaceae-Familie, der Acetobacteraceae-Familie, der Bifidobacteriaceae-Familie, der Comamonadaceae-Familie, der Barnesiellaceae-Familie, der Peptostreptococcaceae-Familie, der Halomonadaceae-Familie, der Rikenellaceae-Familie und der Methylocystaceae-Familie; oder
(x) einem oder mehreren Bakterien, die ausgewählt sind aus der Gruppe bestehend aus der Gattung Ralstonia, der Gattung Rhizobium, der Gattung Morganella, der Gattung Tetragenococcus, der Gattung Exiguobacterium, der Gattung Streptomyces, der Gattung Oribacterium, der Gattung Sporosarcina, der Gattung Jeotgalicoccus, der Gattung Fimbriimonas, der Gattung Enterococcus, der Gattung Porphyromonas, der Gattung Lactococcus, der Gattung Cellulomonas, der Gattung Proteus, der Gattung Granulicatella, der Gattung Acinetobacter, der Gattung Actinomyces, der Gattung Cupriavidus, der Gattung Rhodococcus, der Gattung Fusobacterium, der Gattung Pseudomonas, der Gattung Akkermansia, der Gattung Leptotrichia, der Gattung Methylobacterium, der Gattung Weissella, der Gattung Bacteroides, der Gattung Veillonella, der Gattung Dermacoccus, der Gattung Coprococcus, der Gattung Ruminococcus, der Gattung Trabulsiella, der Gattung Gordonia, der Gattung Lachnospira, der Gattung Klebsiella, der Gattung Enterobacter, der Gattung Faecalibacterium, der Gattung Serratia, der Gattung Bifidobacterium, der Gattung Citrobacter, der Gattung Bilophila, der Gattung Virgibacillus, der Gattung Halomonas, der Gattung Roseburia, der Gattung Comamonas, der Gattung Methylopila und der Gattung Gemella.

4. Verfahren nach Anspruch 3, wobei das Blut Vollblut, Serum oder Plasma ist.

## Revendications

1. Procédé de fourniture d'informations pour le diagnostic du cancer du sein, le procédé consistant à :
(a) extraire de l'ADN de vésicules extracellulaires isolées d'un échantillon sujet ;
(b) réaliser une réaction en chaîne par polymérase (PCR) sur l'ADN extrait en utilisant une paire d'amorces ayant SEQ ID NO: 1 et SEQ ID NO: 2 ; et
(c) comparer une augmentation ou une diminution de la teneur en vésicules extracellulaires dérivées de bactéries de l'échantillon sujet à celle d'un échantillon dérivé d'un individu normal par séquençage d'un produit de la PCR,
dans lequel la comparaison consiste à comparer, entre des échantillons de sang, une augmentation ou diminution de la teneur en vésicules extracellulaires dérivées
(i) d'une ou de plusieurs bactéries sélectionnées à partir du groupe constitué de phylum Fusobacteria et de phylum *Bacteroidetes* ;
(ii) d'une ou de plusieurs bactéries sélectionnées à partir du groupe constitué de la classe Fusobacteriia, la classe *Alphaproteobacteria,* la classe des *chloroplastes,* la classe TM7-3 et la classe *Bacteroidia ;*
(iii) d'une ou de plusieurs bactéries sélectionnées à partir du groupe constitué de l'ordre *Fusobacteriales,* l'ordre *Sphingomonadales,* l'ordre *Neisseriales,* l'ordre *Rhizobiales,* l'ordre *Rhodospirillales,* l'ordre *Actinomycetales,* l'ordre *Bacillales,* l'ordre *Streptophyta,* l'ordre *Caulobacterales,* l'ordre *Pseudomonadales,* l'ordre *Bacteroidales,* l'ordre *Enterobacteriales* et l'ordre *Bifidobacteriales ;*
(iv) d'une ou de plusieurs bactéries sélectionnées à partir du groupe constitué de la famille *Staphylococcaceae,* la famille *Fusobacteriaceae,* la famille *Actinomycetaceae,* la famille *Brevibocterioceae,* la famille *Peptostreptococcaceae,* la famille *Rhizobiaceae,* la famille *Corynebacteriaceae,* la famille *Methylobacteriaceae,* la famille *Propionibacteriaceae,* la famille *Sphingomonadaceae,* famille *Neisseriaceae,* la famille *Flavobacteriaceae,* la famille *Intrasporangiaceae,* la famille *Nocardiaceae,* la famille *Caulobacteraceae,* la famille *Micrococcaceae,* la famille *Oxalobacteraceae,* la famille *Moraxellaceae,* la famille *Pseudomonadaceae,* la famille *Porphyromonodoceae,* la famille *Clostridiaceae,* la famille *Rikenellaceae,* la famille *Veillonellaceae,* la famille *Enterobacteriaceae,* la famille S24-7, la famille *Bacteroidaceae* et la famille *Bifidobacteriaceae ;* ou
(v) d'une ou de plusieurs bactéries sélectionnées à partir du groupe constitué du genre *Hydrogenophilus,* du genre *Staphylococcus,* du genre *Fusobacterium,* du genre *Actinomyces,* du genre *Brevibacterium,* du genre *Granulicatella,* du genre *Neisseria,* du genre *Rothia,* du genre *Corynebacterium,* du genre *Brevundimon,* du genre *Propionibacterium,* du genre *Porphyromonas,* du genre *Sphingomonas,* du genre *Methylobacterium,* du genre *Micrococcus,* du genre *Coprococcus,* du genre *Rhodococcus,* du genre *Cupriavidus,* du genre *Acinetobacter,* du genre *Pseudomonas,* du genre *Enhydrobacter,* du genre *Dialister,* du genre *Tepidimonas,* du genre *Veillonella,* du genre *Phascolarctobacterium,* du genre *Lachnospira,* du genre *Klebsiella,* du genre *Roseburia,* du genre *Parabacteroides,* du genre *Bacteroides,* du genre *Bifidobacterium,* du genre *Megamonas,* et du genre *Enterobacter ;*
ou dans lequel la comparaison consiste à comparer, entre des échantillons d'urine, une augmentation ou diminution de la teneur en vésicules extracellulaires dérivées
(vi) d'une ou de plusieurs bactéries sélectionnées à partir du groupe constitué du phylum *Tenericutes,* du phylum *Armatimonadetes,* du phylum *Cyanobacteria,* du phylum *Verrucomicrobia,* du phylum TM7 et du phylum *Fusobacteria ;*
(vii) d'une ou de plusieurs bactéries sélectionnées à partir du groupe constitué de la classe *Mollicutes,* la classe des *chloroplastes,* la classe *Fimbriimonadia,* la classe TM7-3, la classe *Verrucomicrobiae,* la classe *Saprospirae,* la classe *Fusobacteriia* et la classe 4C0d-2;
(viii) d'une ou de plusieurs bactéries sélectionnées à partir du groupe constitué de l'ordre *Stramenopiles,* l'ordre RF39, l'ordre *Streptophyta,* l'ordre *Fimbriimonadales,* l'ordre *Verrucomicrobiales,* l'ordre *Saprospirales,* l'ordre *Pseudomonadales,* l'ordre *Fusobacteriales,* l'ordre *Burkholderiales,* l'ordre *Bifidobacteriales,* l'ordre *Oceanospirillales* et l'ordre YS2 ;
(ix) d'une ou de plusieurs bactéries sélectionnées à partir du groupe constitué de la famille *Exiguobacteraceae,* la famille *Cellulomonadaceae,* la famille F16, la famille *Fimbriimonadaceae,* la famille *Oxalobacteraceae,* la famille *Streptomycetaceae,* la famille *Carnobacteriaceae,* la famille *Actinomycetaceae,* la famille *Nocardiaceae,* la famille *Peptococcaceae,* la famille *Fusobacteriaceae,* la famille *Mogibacteriaceae,* la famille *Pseudomonadaceae,* la famille *Verrucomicrobiaceae,* la famille *Bradyrhizobiaceae,* la famille *Enterococcaceae,* la famille *Chitinophagaceae,* la famille *Moraxellaceae,* la famille *Rhizobiaceae,* la famille *Ruminococcaceae,* la famille *Bacteroidaceae,* la famille *Dermacoccaceae,* la famille *Gordoniaceae,* la famille *Acetobacteraceae,* la famille *Bifidobacteriaceae,* la famille *Comamonadaceae,* la famille *Barnesiellaceae,* la famille *Peptostreptococcaceae,* la famille *Halomonadaceae,* la famille *Rikenellaceae* et la famille *Methylocystaceae ;* ou
(x) d'une ou de plusieurs bactéries sélectionnées à partir du groupe constitué du genre *Ralstonia,* du genre *Rhizobium,* du genre *Morganella,* du genre *Tetragenococcus,* du genre *Exiguobacterium,* du genre *Streptomyces,* du genre *Oribacterium,* du genre *Sporosarcina,* du genre *Theotgalicoccus,* du genre *Fimbriimonas,* du genre *Enterococcus,* du genre *Porphyromonas,* du genre *Lactococcus,* du genre *Cellulomonas,* du genre *Proteus,* du genre *Granulicatella,* du genre *Acinetobacter,* du genre *Actinomyces,* du genre *Cupriavidus,* du genre *Rhodococcus,* du genre *Fusobacterium,* du genre *Pseudomonas,* du genre *Akkermansia,* du genre *Leptotrichia,* du genre *Methylobacterium,* du genre *Weissella,* du genre *Bacteroides,* du genre *Veillonella,* du genre *Dermacoccus,* du genre *Coprococcus,* du genre *Ruminococcus,* du genre *Trabulsiella,* du genre *Gordon,* du genre *Klebsiella,* du genre *Enterobacter,* du genre *Faecalibacterium,* du genre *Serratia,* du genre *Bifidobacterium,* du genre *Citrobacter,* du genre *Bilophila,* du genre *Virgibacillus,* du genre *Halomonas,* du genre *Roseburia,* du genre *Comamonas,* du genre *Methylopila* et du genre *Gemella.*

2. Procédé selon la revendication 1, dans lequel le sang est du sang total, du sérum ou du plasma.

3. Procédé de diagnostic du cancer du sein, le procédé consistant à :
(a) extraire de l'ADN de vésicules extracellulaires isolées d'un échantillon sujet ;
(b) réaliser une réaction en chaîne par polymérase (PCR) sur l'ADN extrait en utilisant une paire d'amorces ayant SEQ ID NO: 1 et SEQ ID NO: 2 ; et
(c) comparer une augmentation ou une diminution de la teneur en vésicules extracellulaires dérivées de bactéries de l'échantillon sujet à celle d'un échantillon dérivé d'un individu normal par séquençage d'un produit de la PCR ; et
(d) diagnostiquer le cancer du sein en analysant l'augmentation ou la diminution de la teneur en vésicules extracellulaires dérivées de bactéries,
dans lequel la comparaison consiste à comparer, entre des échantillons de sang, une augmentation ou diminution de la teneur en vésicules extracellulaires dérivées
(i) d'une ou de plusieurs bactéries sélectionnées à partir du groupe constitué de phylum Fusobacteria et de phylum *Bacteroidetes ;*
(ii) d'une ou de plusieurs bactéries sélectionnées à partir du groupe constitué de la classe *Fusobacteriia,* la classe *Alphaproteobacteria,* la classe des *chloroplastes,* la classe TM7-3 et la classe *Bacteroidia ;*
(iii) d'une ou de plusieurs bactéries sélectionnées à partir du groupe constitué de l'ordre *Fusobacteriales,* l'ordre *Sphingomonadales,* l'ordre *Neisseriales,* l'ordre *Rhizobiales,* l'ordre *Rhodospirillales,* l'ordre *Actinomycetales,* l'ordre *Bacillales,* l'ordre *Streptophyta,* l'ordre *Caulobacterales,* l'ordre *Pseudomonadales,* l'ordre *Bacteroidales,* l'ordre *Enterobacteriales* et l'ordre *Bifidobacteriales ;*
(iv) d'une ou de plusieurs bactéries sélectionnées à partir du groupe constitué de la famille *Staphylococcaceae,* la famille *Fusobacteriaceae,* la famille *Actinomycetaceae,* la famille *Brevibacteriaceae,* la famille *Peptostreptococcaceae,* la famille *Rhizobiaceae,* la famille *Corynebacteriaceae,* la famille *Methylobacteriaceae,* la famille *Propionibacteriaceae,* la famille *Sphingomonadaceae,* famille *Neisseriaceae,* la famille *Flavobacteriaceae,* la famille *Intrasporangiaceae,* la famille *Nocardiaceae,* la famille *Caulobacteraceae,* la famille *Micrococcaceae,* la famille *Oxalobacteraceae,* la famille *Moraxellaceae,* la famille *Pseudomonadaceae,* la famille *Porphyromonadaceae,* la famille *Clostridiaceae,* la famille *Rikenellaceae,* la famille *Veillonellaceae,* la famille *Enterobacteriaceae,* la famille S24-7, la famille *Bacteroidaceae* et la famille *Bifidobacteriaceae ;* ou
(v) d'une ou de plusieurs bactéries sélectionnées à partir du groupe constitué du genre *Hydrogenophilus,* du genre *Staphylococcus,* du genre *Fusobacterium,* du genre *Actinomyces,* du genre *Brevibacterium,* du genre *Granulicatella,* du genre *Neisseria,* du genre *Rothia,* du genre *Corynebacterium,* du genre *Brevundimon,* du genre *Propionibacterium,* du genre *Porphyromonas,* du genre *Sphingomonas,* du genre *Methylobacterium,* du genre *Micrococcus,* du genre *Coprococcus,* du genre *Rhodococcus,* du genre *Cupriavidus,* du genre *Acinetobacter,* du genre *Pseudomonas,* du genre *Enhydrobacter,* du genre *Dialister,* du genre *Tepidimonas,* du genre *Veillonella,* du genre *Phascolarctobacterium,* du genre *Lachnospira,* du genre *Klebsiella,* du genre *Roseburia,* du genre *Parabacteroides,* du genre *Bacteroides,* du genre *Bifidobacterium,* du genre *Megamonas,* et du genre *Enterobacter ;*
ou dans lequel la comparaison consiste à comparer, entre des échantillons d'urine, une augmentation ou diminution de la teneur en vésicules extracellulaires dérivées
(vi) d'une ou de plusieurs bactéries sélectionnées à partir du groupe constitué du phylum *Tenericutes,* du phylum *Armatimonadetes,* du phylum *Cyanobacteria,* du phylum *Verrucomicrobia,* du phylum TM7 et du phylum *Fusobacteria ;*
(vii) d'une ou de plusieurs bactéries sélectionnées à partir du groupe constitué de la classe *Mollicutes,* la classe des *chloroplastes,* la classe *Fimbriimonadia,* la classe TM7-3, la classe *Verrucomicrobiae,* la classe *Saprospirae,* la classe *Fusobacteriia* et la classe 4C0d-2;
(viii) d'une ou de plusieurs bactéries sélectionnées à partir du groupe constitué de l'ordre *Stramenopiles,* l'ordre RF39, l'ordre *Streptophyta,* l'ordre *Fimbriimonadales,* l'ordre *Verrucomicrobiales,* l'ordre *Saprospirales,* l'ordre *Pseudomonadales,* l'ordre *Fusobacteriales,* l'ordre *Burkholderioles,* l'ordre *Bifidobacteriales,* l'ordre *Oceanospirillales* et l'ordre YS2 ;
(ix) d'une ou de plusieurs bactéries sélectionnées à partir du groupe constitué de la famille *Exiguobacteraceae,* la famille *Cellulomonadaceae,* la famille F16, la famille *Fimbriimonadaceae,* la famille *Oxalobacteraceae,* la famille *Streptomycetaceae,* la famille *Carnobacteriaceae,* la famille *Actinomycetaceae,* la famille *Nocardiaceae,* la famille *Peptococcaceae,* la famille *Fusobacteriaceae,* la famille *Mogibacteriaceae,* la famille *Pseudomonadaceae,* la famille *Verrucomicrobiaceae,* la famille *Bradyrhizobiaceae,* la famille *Enterococcaceae,* la famille *Chitinophagaceae,* la famille *Moraxellaceae,* la famille *Rhizobiaceae,* la famille *Ruminococcaceae,* la famille *Bacteroidaceae,* la famille *Dermacoccaceae,* la famille *Gordoniaceae,* la famille *Acetobacteraceae,* la famille *Bifidobacteriaceae,* la famille *Comamonadaceae,* la famille *Bornesiellaceace,* la famille *Peptostreptococcaceae,* la famille *Halomonadaceae,* la famille *Rikenellaceae* et la famille *Methylocystaceae ;* ou
(x) d'une ou de plusieurs bactéries sélectionnées à partir du groupe constitué du genre *Ralstonia,* du genre *Rhizobium,* du genre *Morganella,* du genre *Tetragenococcus,* du genre *Exiguobacterium,* du genre *Streptomyces,* du genre *Oribacterium,* du genre *Sporosarcina,* du genre *Theotgalicoccus,* du genre *Fimbriimonas,* du genre *Enterococcus,* du genre *Porphyromonas,* du genre *Lactococcus,* du genre *Cellulomonas,* du genre *Proteus,* du genre *Granulicatella,* du genre *Acinetobacter,* du genre *Actinomyces,* du genre *Cupriavidus,* du genre *Rhodococcus,* du genre *Fusobacterium,* du genre *Pseudomonos,* du genre *Akkermonsio,* du genre *Leptotrichia,* du genre *Methylobacterium,* du genre *Weissella,* du genre *Bocteroides,* du genre *Veillonella,* du genre *Dermococcus,* du genre *Coprococcus,* du genre *Ruminococcus,* du genre *Trabulsiella,* du genre *Gordon,* du genre *Klebsiella,* du genre *Enterobocter,* du genre *Faecalibacterium,* du genre *Serratia,* du genre *Bifidobacterium,* du genre *Citrobocter,* du genre *Bilophila,* du genre *Virgibacillus,* du genre *Halomonas,* du genre *Roseburio,* du genre *Comamonas,* du genre *Methylopila* et du genre *Gemella.*

4. Procédé selon la revendication 3, dans lequel le sang est du sang total, du sérum ou du plasma.


<110> MD Healthcare Inc.
<120> Method for diagnosis of breast cancer using analysis of microbial metagenome
<130> MPCT17-093
<150> 10-2016-0179247
   <151> 2016-12-26
<150> 10-2017-0074046
   <151> 2017-06-13
<160> 2
<170> KoPatentIn 3.0
<210> 1
   <211> 50
   <212> DNA
   <213> Artificial Sequence
<220>
   <223> 16S_V3_F
<400> 1
   tcgtcggcag cgtcagatgt gtataagaga cagcctacgg gnggcwgcag 50
<210> 2
   <211> 55
   <212> DNA
   <213> Artificial Sequence
<220>
   <223> 16S_V4_R
<400> 2
   gtctcgtggg ctcggagatg tgtataagag acaggactac hvgggtatct aatcc 55